# EUROPÄISCHE PATENTSCHRIFT

(11) **EP 0 512 349 B1**
(45) Veröffentlichungstag und Bekanntmachung des Hinweises auf die Patenterteilung: **14.02.1996**
(21) Anmeldenummer: 92107130.4
(22) Anmeldetag: 27.04.1992
(51) Int. Cl.: C07D 333/70, A61K 31/38, A01N 43/12

(54) **Benzothiophen-2-carboxamid-S,S-dioxide**
Benzothiophene-2-carboxamide-S,S-dioxides
S,S-Dioxydes de benzothiophène-2-carboxamide

(30) Priorität: 09.05.1991 DE 4115184
(43) Veröffentlichungstag der Anmeldung: 11.11.1992
(73) Patentinhaber: BAYER AG, D-51368 Leverkusen (DE)
(72) Erfinder: Elbe, Hans-Ludwig, Dr., W-5600 Wuppertal 1 (DE); Berg, Dieter, Prof. Dr., W-5600 Wuppertal 1 (DE); Dehne, Heinz-Wilhelm, Dr., W-4019 Monheim (DE); Dutzmann, Stefan, Dr., W-4010 Hilden 1 (DE); Ludwig, Georg-Wilhelm, Dr., W-4150 Krefeld (DE); Plempel, Manfred, Dr., W-5657 Haan (DE)

(56) Entgegenhaltungen:
- EP-A- 0 146 243
- DE-A- 3 832 846
- DE-A- 3 832 848
- GB-A- 2 193 961

## Beschreibung

Die Erfindung betrifft neue Benzothiophen-2-carboxamid-S,S-dioxide, ein Verfahren zu ihrer Herstellung und ihre Verwendung zur Bekämpfung von Schädlingen auf verschiedenen Gebieten, z.B. im Pflanzenschutz und im Materialschutz.

Es ist bekannt, daß bestimmte Benzothiophen-2-carboxamid-S,S-dioxide, wie beispielsweise die Verbindung 3-Chlor-N-(1-phenylethyl)-benzothiophen-2-carboxamid-S,S-dioxid fungizide Eigenschaften besitzen (vergleiche z. B. DE- A 38 32 848).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin ist bekannt, daß bestimmte Benzothiophen-2-carboxamid-S,S-dioxide, wie beispielsweise die Verbindung 3-(1-Imidazolyl)-N-(1-phenylethyl)-benzothiophen-2-carboxamid-S,S-dioxid oder die Verbindung 3-Methylthio-N-(1-phenylethyl)-benzothiophen-2-carboxamid-S,S-dioxid eine gute antimykotische Wirksamkeit besitzen (vergleiche z.B. DE-A 38 32 848).

Die Wirksamkeit dieser vorbekannten Verbindungen ist jedoch nicht in allen Indikationen völlig zufriedenstellend.

Außerdem sind bestimmte Benzothiophen-2-carboxamid-Derivate bekannt (vergleiche z.B. EP-A 146 243 oder GB-A 21 93 961), über deren Wirksamkeit bisher nichts bekannt ist.

Ferner geht aus der DE-A 3 832 846 hervor, daß bestimmte Benzothiophen-2-carboxamid-S,S-dioxide, die in der 3-Position einen zusätzlichen Substituenten enthalten, pharmazeutische Eigenschaften besitzen und zur Behandlung von Mykosen geeignet sind. Entsprechende Stoffe, die in der 3-Position unsubstituiert sind, werden jedoch nicht erwähnt.

Es wurden neue Benzothiophen-2-carboxamid-S,S-dioxide der Formel 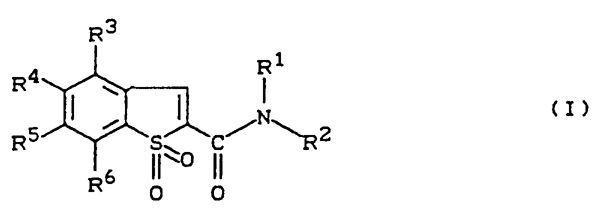 in welcher
- R¹: für geradkettiges oder verzweigtes Alkyl mit 1 bis 20 Kohlenstoffatomen, für jeweils geradkettiges oder verzweigtes Halogenalkyl, Cyanalkyl, Hydroxyalkyl, Alkoxyalkyl oder Alkoxycarbonylalkyl mit jeweils 1 bis 8 Kohlenstoffatomen in den einzelnen Alkylteilen, für geradkettiges oder verzweigtes Alkenyl mit 2 bis 12 Kohlenstoffatomen, für geradkettiges oder verzweigtes Alkinyl mit 2 bis 12 Kohlenstoffatomen oder für jeweils gegebenenfalls im Cycloalkylteil ein- bis sechsfach, gleich oder verschieden substituiertes Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht, wobei als Cycloalkylsubstituenten jeweils infrage kommen: Halogen, jeweils geradkettiges oder verzweigtes Alkyl mit 1 bis 4 Kohlenstoffatomen oder jeweils geradkettiges oder verzweigtes Halogenalkyl mit 1 bis 4 Kohlenstoffatomen und 1 bis 9 gleichen oder verschiedenen Halogenatomen; außerdem für jeweils gegebenenfalls im Arylteil ein- bis fünffach, gleich oder verschieden substituiertes Arylalkyl, Arylalkenyl, Arylalkinyl oder Aryl mit jeweils 6 oder 10 Kohlenstoffatomen im Arylteil und gegebenenfalls bis zu 12 Kohlenstoffatomen im jeweils geradkettigen oder verzweigten Alkyl-bzw. Alkenyl- oder Alkinylteil Steht, wobei als Arylsubstituenten jeweils infrage kommen: Halogen, Cyano, Nitro, Formylamido, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkylcarbonyl, Alkoxycarbonyl, Aminocarbonyl, N-Alkylaminocarbonyl, N,N-Dialkylaminocarbonyl, Alkylcarbonylamino, N-Alkyl-Alkylcarbonylamino, N-Alkyl-Formylcarbonylamino oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,
- R: für Wasserstoff oder für gegebenenfalls einfach oder mehrfach, gleich oder verschieden substituiertes, geradkettiges oder verzweigtes Alkyl mit 1 bis 6 Kohlenstoffatomen steht, wobei als Substituenten infrage kommen: Hydroxy, Halogen, Cyano sowie jeweils geradkettiges oder verzweigtes Alkoxy, Alkoxycarbonyl oder Dialkylamino mit jeweils 1 bis 6 Kohlenstoffatomen in den einzelnen Alkylteilen oder
- R¹ und R: gemeinsam mit dem Stickstoffatom, an welches sie gebunden sind, für einen gegebenenfalls einfach oder mehrfach, gleich oder verschieden substituierten, gesättigten 5- bis 7-gliedrigen Heterocyclus stehen, der gegebenenfalls 1 oder 2 weitere Heteroatome, insbesondere Stickstoff, Sauerstoff und/oder Schwefel, enthalten kann, wobei als Substituenten infrage kommen: Halogen, jeweils geradkettiges oder verzweigtes Alkyl, Alkoxy oder Alkoxycarbonyl mit jeweils 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen und
- R³, R⁴, R⁵ und R⁶: unabhängig voneinander jeweils für Wasserstoff, Halogen, Cyano, Nitro oder für jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 13 gleichen oder verschiedenen Halogenatomen stehen,
gefunden.

Weiterhin wurde gefunden, daß man die neuen Benzothiophen-2-carboxamid-S,S-dioxide der Formel (I) erhält, wenn man Benzothiophen-2-carboxamide der Formel 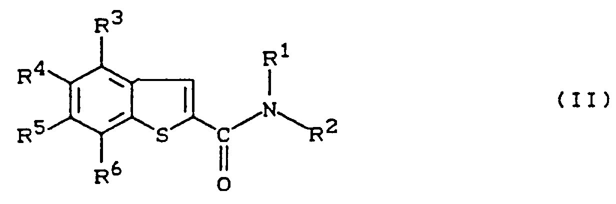 in welcher
R¹, R, R³, R⁴, R⁵ und R⁶ die oben angegebene Bedeutung haben,
mit einem Oxidationsmittel gegebenenfalls in Gegenwart eines Verdünnungsmittels und gegebenenfalls in Gegenwart eines Reaktionshilfsmittels umsetzt.

Schließlich wurde gefunden, daß die neuen Benzothiophen-2-carboxamid-S,S-dioxide der Formel (I) eine gute Wirksamkeit gegen Schädlinge auf den verschiedensten Gebieten besitzen, so gegen Pilze auf dem Pflanzenschutzgebiet und gegen Mikroorganismen im Materialschutz.

Überraschenderweise zeigen die erfindungsgemäßen Benzothiophen-2-carboxamid-S,S-dioxide der Formel (I) eine bessere fungizide Wirksamkeit als die aus dem Stand der Technik bekannten Benzothiophen-2-carboxamid-S,S-dioxide, wie beispielsweise die Verbindung 3-Chlor-N-(1-phenylethyl)-benzothiophen-2-carboxamid-S,S-dioxid, welches chemisch und wirkungsmäßig naheliegende Verbindungen sind.

Die erfindungsgemäßen Benzothiophen-2-carboxamid-S,S-dioxide sind durch die Formel (I) allgemein definiert. Bevorzugt sind Verbindungen der Formel (I), bei welchen
- R¹: für geradkettiges oder verzweigtes Alkyl mit 1 bis 18 Kohlenstoffatomen, für jeweils geradkettiges oder verzweigtes Halogenalkyl, Cyanalkyl, Hydroxyalkyl, Alkoxyalkyl oder Alkoxycarbonylalkyl mit jeweils 1 bis 4 Kohlenstoffatomen in den einzelnen Alkylteilen, für geradkettiges oder verzweigtes Alkenyl mit 2 bis 8 Kohlenstoffatomen, für geradkettiges oder verzweigtes Alkinyl mit 2 bis 8 Kohlenstoffatomen oder für jeweils gegebenenfalls im Cycloalkylteil ein- bis vierfach, gleich oder verschieden substituiertes Cycloalkylalkyl oder Cycloalkyl mit jeweils 3 bis 6 Kohlenstoffatomen im Cycloalkylteil und gegebenenfalls 1 bis 4 Kohlenstoffatomen im geradkettigen oder verzweigten Alkylteil steht, wobei als Cycloalkylsubstituenten jeweils infrage kommen: Fluor, Chlor, Brom, Methyl, Ethyl, n- oder i-Propyl, n-, i-, s- oder t-Butyl, Chlormethyl, Dichlormethyl oder Trifluormethyl; außerdem für jeweils gegebenenfalls im Arylteil ein- bis fünffach, gleich oder verschieden substituiertes Arylalkyl, Arylalkenyl, Arylalkinyl oder Aryl mit jeweils 6 oder 10 Kohlenstoffatomen im Arylteil und gegebenenfalls bis zu 8 Kohlenstoffatomen im jeweils geradkettigen oder verzweigten Alkyl- bzw. Alkenyl- oder Alkinylteil steht, wobei als Arylsubstituenten jeweils infrage kommen:
Halogen, Hydroxy, Cyano, Nitro, Formylamido, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkylcarbonyl, Alkoxycarbonyl, Aminocarbonyl, N-Alkylaminocarbonyl, N,N-Dialkylaminocarbonyl, Alkylcarbonylamino, N-Alkyl-Alkylcarbonylamino, N-Alkyl-Formylcarbonylamino oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,
- R: für Wasserstoff oder für gegebenenfalls einfach oder mehrfach, gleich oder verschieden substituiertes, geradkettiges oder verzweigtes Alkyl mit 1 bis 4 Kohlenstoffatomen steht, wobei als Substituenten infrage kommen: Hydroxy, Halogen, Cyano sowie jeweils geradkettiges oder verzweigtes Alkoxy, Alkoxycarbonyl oder Dialkylamino mit jeweils 1 bis 4 Kohlenstoffatomen in den einzelnen Alkylteilen oder
- R¹ und R: gemeinsam mit dem Stickstoffatom, an welches sie gebunden sind, für einen gegebenenfalls ein-bis vierfach, gleich oder verschieden substituierten Heterocyclus der Formel 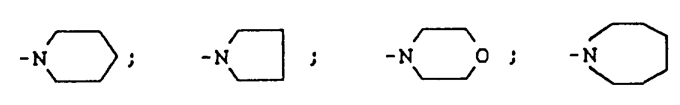 oder 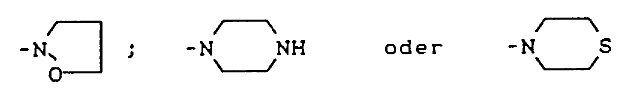 stehen, wobei als Substituenten jeweils infrage kommen: Fluor, Chlor, Brom, Methyl, Ethyl, n- oder i-Propyl, Methoxy, Ethoxy, Methoxycarbonyl, Ethoxycarbonyl, Chlormethyl, Trichlormethyl, Dichlormethyl oder Trifluormethyl und
- R³, R⁴, R⁵ und R⁶: unabhängig voneinander jeweils für Wasserstoff, Halogen, Cyano, Nitro oder für jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen stehen.

Besonders bevorzugt sind Verbindungen der Formel (I), bei welchen
- R¹: für Methyl, Ethyl, n- oder i-Propyl, n-, i-, s-oder t-Butyl, n- oder i-Pentyl, n- oder i-Hexyl, n- oder i-Heptyl, n- oder i-Octyl, n- oder i-Nonyl, n- oder i-Decyl, n- oder i-Dodecyl, n- oder i-Octadecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, Chlormethyl, Brommethyl, Chlorethyl, Bromethyl, Chlorpropyl, Brompropyl, Cyanomethyl, Cyanoethyl, Cyanopropyl, Hydroxymethyl, Hydroxyethyl, Hydroxypropyl, Methoxymethyl, Methoxyethyl, Methoxypropyl, Ethoxymethyl, Ethoxyethyl, Ethoxypropyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl; außerdem für jeweils gegebenenfalls im Cycloalkylteil ein- bis vierfach, gleich oder verschieden durch Fluor, Chlor, Brom, Methyl, Ethyl, n- oder 1-Propyl, Chlormethyl, Dichlormethyl oder Trifluormethyl substituiertes Cyclopropyl, Cyclopropylmethyl, Cyclopropylethyl, Cyclopropylpropyl, Cyclopentyl, Cyclopentylmethyl, Cyclopentylethyl, Cyclopentylpropyl, Cyclohexyl, Cyclohexylmethyl, Cyclohexylethyl oder Cyclohexylpropyl steht; außerdem für jeweils gegebenenfalls im Arylteil ein- bis dreifach, gleich oder verschieden substituiertes Phenylalkyl, Phenylalkenyl, Phenylalkinyl, Phenyl oder Naphthyl mit jeweils gegebenenfalls bis zu 6 Kohlenstoffatomen im geradkettigen oder verzweigten Alkyl- bzw. Alkenyl- oder Alkinylteil steht, wobei als Arylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methylcarbonyl, Ethylcarbonyl, Methoxycarbonyl, Ethoxycarbonyl, Aminocarbonyl, N-Methylaminocarbonyl, N,N-Dimethylaminocarbonyl, N-Ethylaminocarbonyl, N,N-Diethylaminocarbonyl, N-Formylamino, N-Acetylamino, N-Methyl-N-Formylamino, N-Methyl-N-Acetyl-amino, N-Ethyl-N-Formylamino, N-Ethyl-N-Acetyl-amino, Methoximinomethyl, Methoximinoethyl, Ethoximinomethyl, Ethoximinoethyl oder gegebenenfalls ein-bis dreifach, gleich oder verschieden durch Fluor, Chlor, Brom, Methyl und/oder Ethyl substituiertes Phenyl,
- R: für Wasserstoff, Methyl, Ethyl, n- oder i-Propyl, n-, i-, s- oder t-Butyl, n- oder i-Pentyl, n- oder i-Hexyl, Hydroxymethyl, Hydroxyethyl, Hydroxypropyl, Chlormethyl, Brommethyl, Chlorethyl, Bromethyl, Chlorpropyl, Brompropyl, Cyanomethyl, Cyanoethyl, Cyanopropyl, Hydroxymethyl, Hydroxyethyl, Hydroxypropyl, Methoxymethyl, Methoxyethyl, Methoxypropyl, Ethoxymethyl, Ethoxyethyl, Ethoxypropyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dimethylaminomethyl, Diethylaminomethyl, Dipropylaminomethyl, Dimethylaminoethyl, Diethylaminoethyl, Dipropylaminoethyl, Dimethylaminopropyl, Diethylaminopropyl oder Dipropylaminopropyl steht oder
- R¹ und R: gemeinsam mit dem Stickstoffatom, an welches sie gebunden sind, für einen gegebenenfalls einfach, zweifach oder dreifach durch Methyl und/oder Ethyl, Methoxycarbonyl und/oder Ethoxycarbonyl substituierten Heterocyclus der Formel 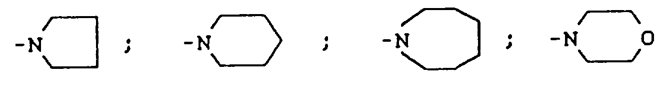 oder 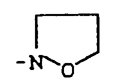 stehen und
- R³, R⁴, R⁵ und R⁶: unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio stehen.

Im einzelnen seien die bei den Herstellungsbeispielen aufgeführten Verbindungen genannt.

Verwendet man beispielsweise Benzothiophen-2-carbonsäureanilid als Ausgangsverbindung und Wasserstoffperoxid als Oxidationsmittel, so läßt sich der Reaktionsablauf des erfindungsgemäßen Verfahrens durch das folgende Formelschema darstellen:

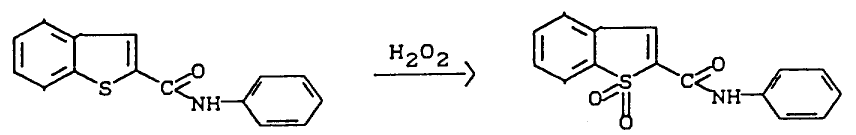

Die zur Durchführung des erfindungsgemäßen Verfahrens als Ausgangsstoffe benötigten Benzothiophen-2-carboxamide sind durch die Formel (II) allgemein definiert. In dieser Formel (II) stehen R¹, R, R³, R⁴, R⁵ und R⁶ vorzugsweise für diejenigen Reste, die bereits im Zusammenhang mit der Beschreibung der erfindungsgemäßen Verbindungen der Formel (I) als bevorzugt für diese Substituenten genannt wurden.

Die Benzothiophen-2-carboxamide der Formel (II) sind bekannt oder erhältlich in Analogie zu bekannten Verfahren (vergleiche z. B. EP-A 374 048; EP-A 253 650; Ind. J. Chem. Sect. B, 23B, 38-41 [1984]; Tetrahedron 34, 3545-3551 [1978]; Collect. Czech. Chem. Commun. 51, 2002-2012 [1986]; J, org. Chem. 40, 3037-3045 [1975]; Liebigs Ann. Chem. 760, 37-87 [1972] sowie die Herstellungsbeispiele).

Als Oxidationsmittel zur Durchführung des erfindungsgemäßen Verfahrens kommen alle üblicherweise für Schwefeloxidationen verwendbaren Oxidationsmittel infrage. Vorzugsweise verwendet man Wasserstoffperoxid oder organische Persäuren, wie beispielsweise Peressigsäure, 4-Nitroperbenzoesäure oder 3-Chlorperbenzoesäure oder auch anorganische Oxidationsmittel, wie Periodsäure, Kaliumpermanganat oder Chromsäure.

Als Verdünnungsmittel zur Durchführung des erfindungsgemäßen Verfahrens kommen in Abhängigkeit vom verwendeten Oxidationsmittel anorganische oder organische Lösungsmittel infrage. Vorzugsweise verwendet man Alkohole, wie beispielsweise Methanol oder Ethanol oder deren Gemische mit Wasser oder reines Wasser; Säuren, wie beispielsweise Essigsäure, Acetanhydrid oder Propionsäure oder dipolar aprotische Lösungsmittel, wie Acetonitril, Aceton, Essigsäureethylester oder Dimethylformamid sowie auch gegebenenfalls halogenierte Kohlenwasserstoffe, wie Benzin, Benzol, Toluol, Hexan, Cyclohexan, Petrolether, Dichlormethan, Dichlorethan, Chloroform, Tetrachlorkohlenstoff oder Chlorbenzol.

Das erfindungsgemäße Verfahren kann gegebenenfalls in Gegenwart eines Reaktionshilfsmittels durchgeführt werden. Als solche kommen alle üblicherweise verwendbaren organischen oder anorganischen Säurebindemittel infrage. Vorzugsweise verwendet man Erdalkalimetall- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren kann gegebenenfalls auch in Gegenwart eines geeigneten Katalysators durchgeführt werden. Als solche kommen alle üblicherweise für derartige Schwefeloxidationen verwendbaren Katalysatoren infrage. Vorzugsweise verwendet man Schwermetallkataly satoren; beispielhaft genannt sei in diesem Zusammenhang Ammoniummolybdat.

Die Reaktionstemperaturen können bei der Durchführung des erfindungsgemäßen Verfahrens in einem größeren Bereich variiert werden. Im allgemeinen arbeitet man bei Temperaturen zwischen -30°C und +100°C, vorzugsweise bei Temperaturen zwischen 0°C und +80°C.

Zur Durchführung des erfindungsgemäßen Verfahrens setzt man pro Mol an Benzothiophen-2-carboxamid der Formel (II) im allgemeinen 2,0 bis 10,0 Mol, vorzugsweise 2,0 bis 5,0 Mol an Oxidationsmittel, gegebenenfalls 1,0 bis 1,5 Mol, vorzugsweise 1,0 bis 1,3 Mol an als Reaktionshilfsmittel verwendeter Base und gegebenenfalls 0,001 bis 1,0 Mol, vorzugsweise 0,005 bis 0,05 Mol an Katalysator ein.
Die Reaktionsdurchführung, Aufarbeitung und Isolierung der Reaktionsprodukte erfolgt nach bekannten Verfahren (vergleiche hierzu die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind u.a. für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis; Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis; Pyrenophora-Arten, wie beispielsweise Pyrenophora teres oder P. graminea
   (Konidienform: Drechslera, Syn: Helminthosporium); Cochliobolus-Arten, wie beispielsweise Cochliobolus sativus
   (Konidienform: Drechslera, Syn: Helminthosporium); Uromyces-Arten, wie beispieisweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Getreidekrankheiten, wie beispielsweise gegen den Erreger des echten Getreidemehltaues (Erysiphe graminis) oder gegen den Erreger der Netzfleckenkrankheit der Gerste (Pyrenophora teres) oder gegen den Erreger der Braunfleckenkrankheit an Gerste oder Weizen (Cochliobolus sativus) oder gegen den Erreger der Braunspelzigkeit des Weizens (Leptosphaeria nodorum) oder zur Bekämpfung von Krankheiten im Obst- und Gemüseanbau, wie beispielsweise gegen den Erreger des Apfelschorfes (Venturia inaequalis) oder gegen Oomyceten oder zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) einsetzen. Daneben besitzen die erfindungsgemäßen Wirkstoffe eine breite in vitro-Wirksamkeit.

Die erfindungsgemäßen Wirkstoffe zeichnen sich neben der oben angeführten Wirksamkeit gegen pflanzenpathogene Mikroorganismen durch eine starke mikrobizide Wirkung gegen ein breites Spektrum von für den Materialschutz relevanten Mikroorganismen aus. Insofern eignen sich die erfindungsgemäßen Substanzen vorzüglich zum Schutz technischer Materialien.

Technische Materialien in diesem Sinne sind nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasser kreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartons, Leder, Holz, Anstrichmittel, Kunststoffartikel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet können die Wirkstoffe in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden flüssigen Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel könnten z. B. auch organische Lösungsmittel, wie beispielsweise Alkohole, als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene wie 1,2-Dichlorethan oder Methylenchlorid, aliphatische Kohlenwasserstoffe, wie Cyclohexan oder Paraffine, z. B. Benzin oder andere Erdölfraktionen, Alkohole, wie Ethanol, Isopropanol, Butanol, Benzylalkohol oder Glycol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen infrage: z. B natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- undloder schaumerzeugende Mittel kommen infrage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen infrage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z. B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die erfindungsgemäßen Wirkstoffe können bei der Anwendung als Materialschutzmittel auch in Mischung mit anderen bekannten Wirkstoffen vorliegen. Beispielsweise seien die folgenden Wirkstoffe genannt: Benzylalkoholmono(oder-poly)hemiformal und andere formaldehydabspaltende Verbindungen, Benzimidazolyl-methylcarbamate, Tetramethyldiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, Phenolderivate, wie 2-Phenylphenol, (2,2'-Dihydroxy-5,5'-dichlor)-diphenylmethan, 3-Methyl-4-chlorphenol und 2-Thiocyanatomethylthiobenzthiazol, N-Trihalogenmethylthioverbindungen wie Folpet, Fluorfolpet und Dichlofluanid, Azolfungizide wie Triadimefon, Triadimenol, Bitertanol, Tebuconazole, Propiconazole, Azaconazole, Jodpropargylderivate, wie z.B. Jodpropargylbutylcarbamat (IPBC) und Jodpropargylphenylcarbamat, Isothiazolinonverbindungen wie Kathon sowie quartäre Ammoniumverbindungen wie Benzalkoniumchlorid. Ebenfalls können Mischungen der erfindungsgemäß zu verwendenden Substanzen mit bekannten Insektiziden Anwendung finden. Beispielhaft seien hier genannte Organophosphorverbindungen wie Chlorpyriphos oder Phoxim, Carbamate wie Aldicarb, Carbosulfan oder Propoxur oder Pyrethoide wie Permethrin, Cyfluthrin, Cypermethrin, Deltamethrin oder Fenvalerat.

Ebenso kommen als Mischpartner Algizide, Molluskizide sowie Wirkstoffe gegen "sea animals", die sich auf Schiffsbodenanstrichen ansiedeln in Frage.

### Herstellungsbeispiele:

### Beispiel 1:

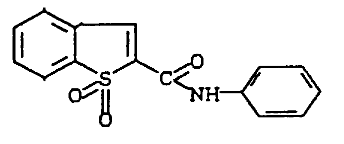

6 g (0,0237 Mol) Benzothiophen-2-carbonsäureanilid-und 9,3 g (0,0947 Mol) 35prozentige wässrige Wasserstoffperoxidlösung werden in 47 ml Eisessig gelöst und 20 Stunden bei 50 °C gerührt. Zur Aufarbeitung wird Wasser zugegeben, ausgefallener Niederschlag abgesaugt, zweimal mit Wasser gewaschen und anschließend getrocknet.

Man erhält 5,5 g (82 % der Theorie) an Benzothiophen-2-carbonsäureanilid-S,S-dioxid vom Schmelzpunkt >200 °C.

### Herstellung der Ausgangsverbindung:

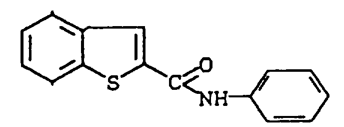

Zu 8 g (0,0407 Mol) Benzothiophen-2-carbonsäurechlorid in 150 ml Toluol gibt man unter Rühren bei Raumtemperatur 7,6 g (0,0814 Mol) Anilin, rührt nach beendeter Zugabe 5 Stunden bei 50 °C, gibt dann die Reaktionsmischung in Wasser, saugt ausgefallenen Niederschlag ab, wäscht zweimal mit Wasser nach und trocknet.

Man erhält 7,9 g (77 % der Theorie) an Benzothiophen-2-carbonsäureanilid vom Schmelzpunkt 188 °C.

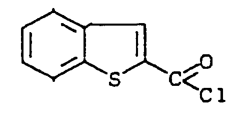

Zu 57,5 g (0,324 Mol) Benzothiophen-2-carbonsäure in 600 ml Chlorbenzol gibt man bei Rückflußtemperatur tropfenweise unter Rühren 57,8 g (0,468 Mol) Thionylchlorid und rührt nach beendeter Zugabe solange bei Rückflußtemperatur bis keine weitere Gasentwicklung mehr zu beobachten ist. Zur Aufarbeitung läßt man die Reaktionsmischung auf Raumtemperatur abkühlen, engt dann im Vakuum ein, verrührt den Rückstand mit n-Hexan, saugt ausgefallenen Niederschlag ab und trocknet ihn.

Man erhält 57,4 g (90 % der Theorie) an Benzothiophen-2-carbonsäurechlorid, welches ohne zusätzliche Reinigung weiter umgesetzt werden kann.

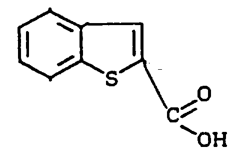

84 g (0,438 Mol) Benzothiophen-2-carbonsäuremethylester (vergleiche z. B. J. Org. Chem. 37, 3224 [1972]) in 218 ml Ethanol und 36,8 g (0,656 Mol) Kaliumhydroxid in 100 ml Wasser werden zusammengegeben und 4 Stunden bei Rückflußtemperatur gerührt. Zur Aufarbeitung läßt man die Reaktionsmischung abkühlen, destilliert dann das Ethanol im Vakuum ab, wäscht den wässrigen Rückstand einmal mit Diethylether, säuert anschließend mit verdünnter Salzsäure an, saugt ausgefallenen Niederschlag ab, wäscht einmal mit Wasser und trocknet.

Man erhält 60,1 g (77 % der Theorie) an Benzothiophen-2-carbonsäure vom Schmelzpunkt >220 °C.

In entsprechender Weise und gemäß den allgemeinen Angaben zur Herstellung erhält man die folgenden Benzothiophen-2-carboxamid-S,S-dioxide der Formel (I):

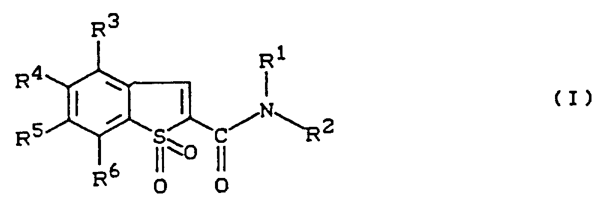

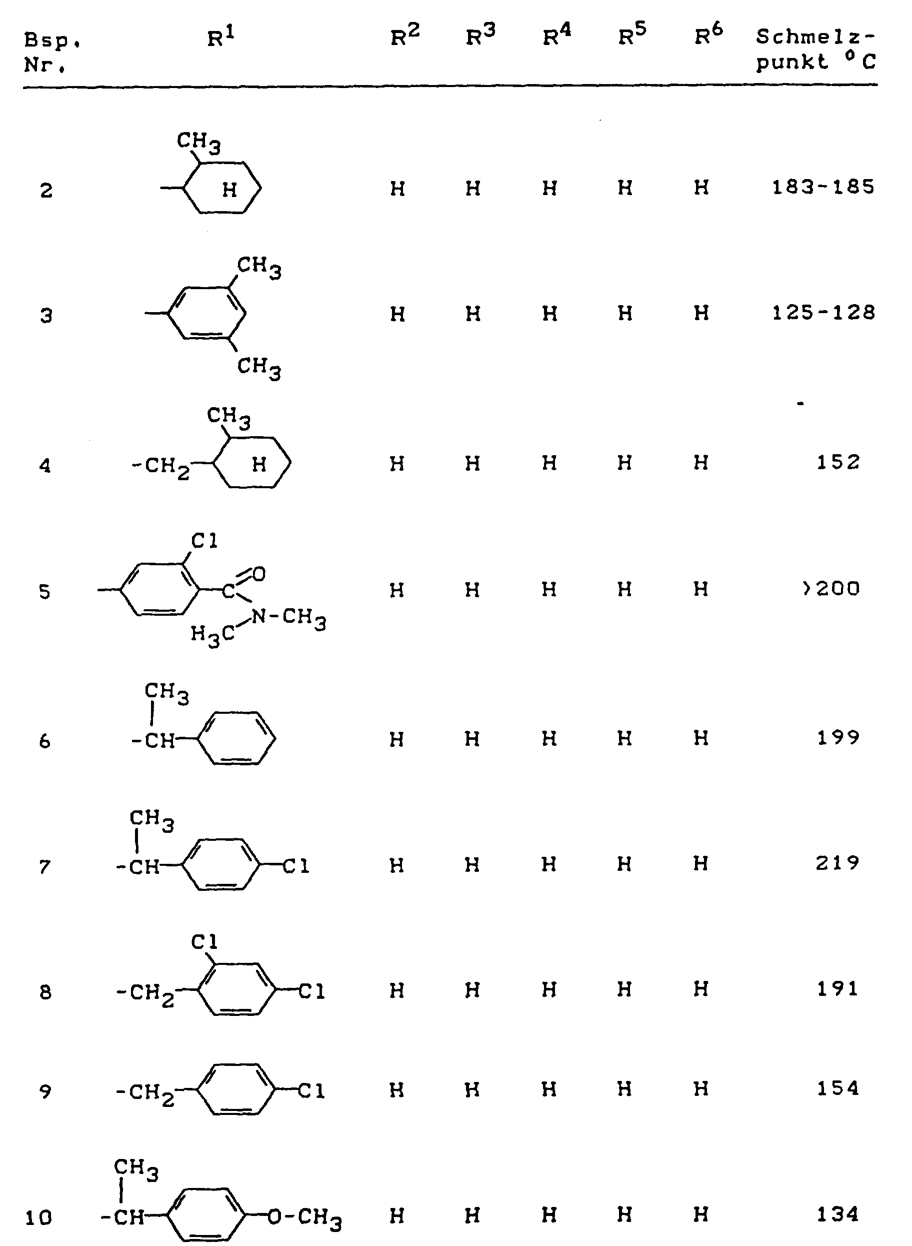

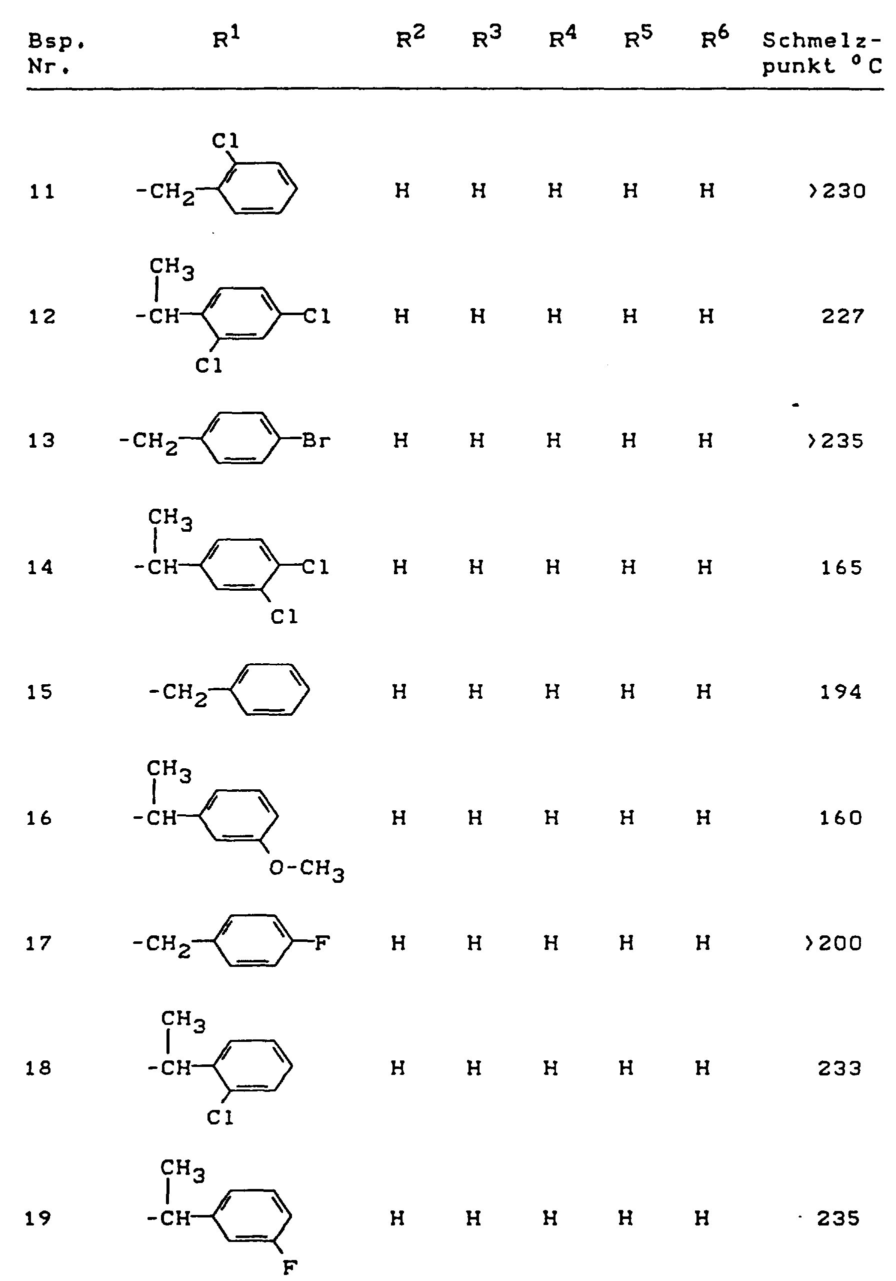

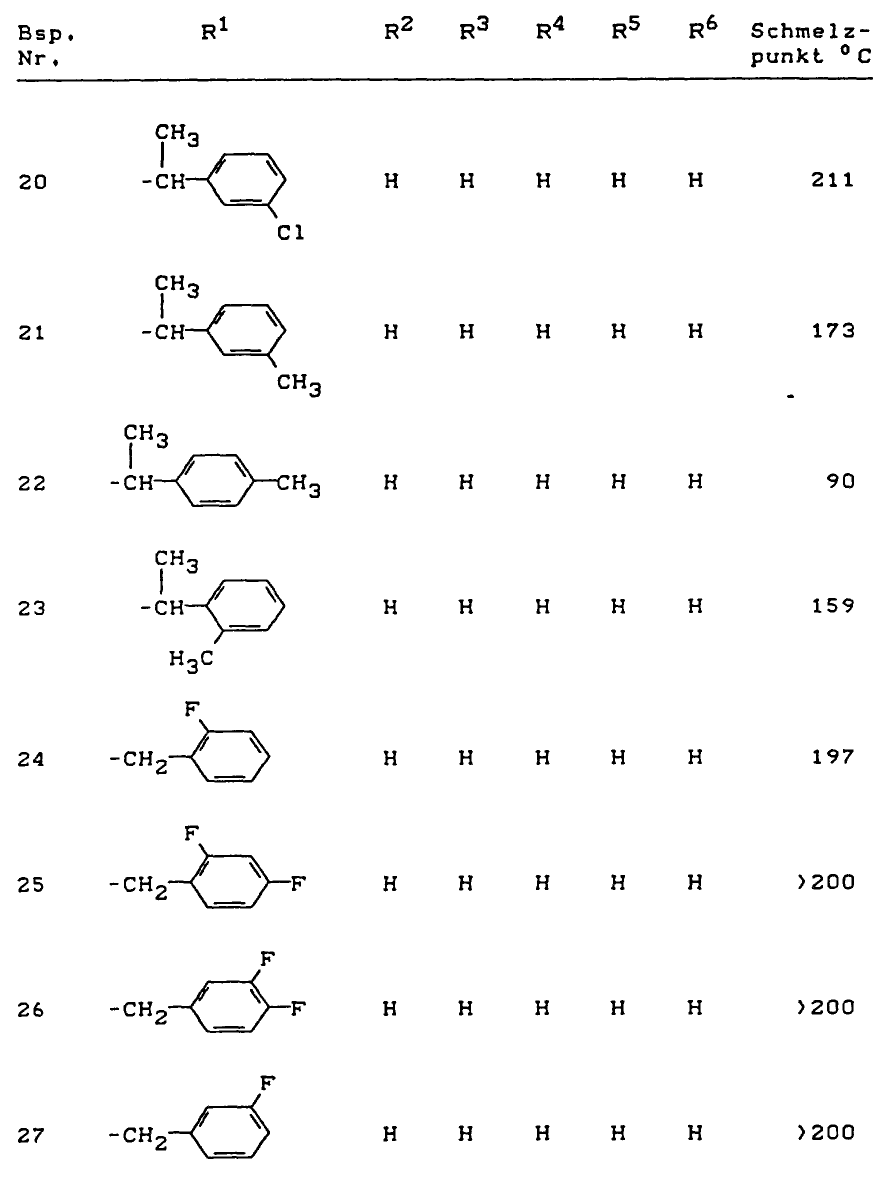

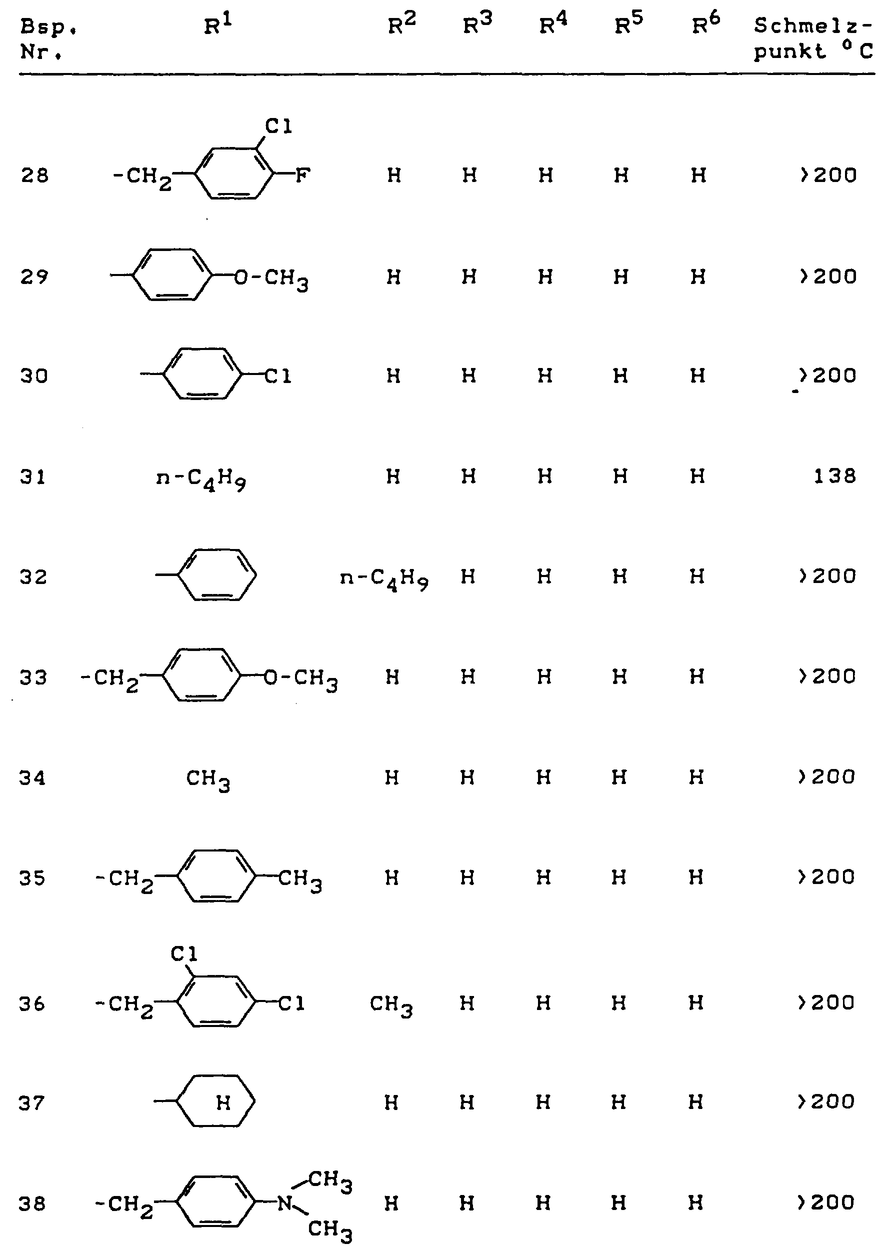

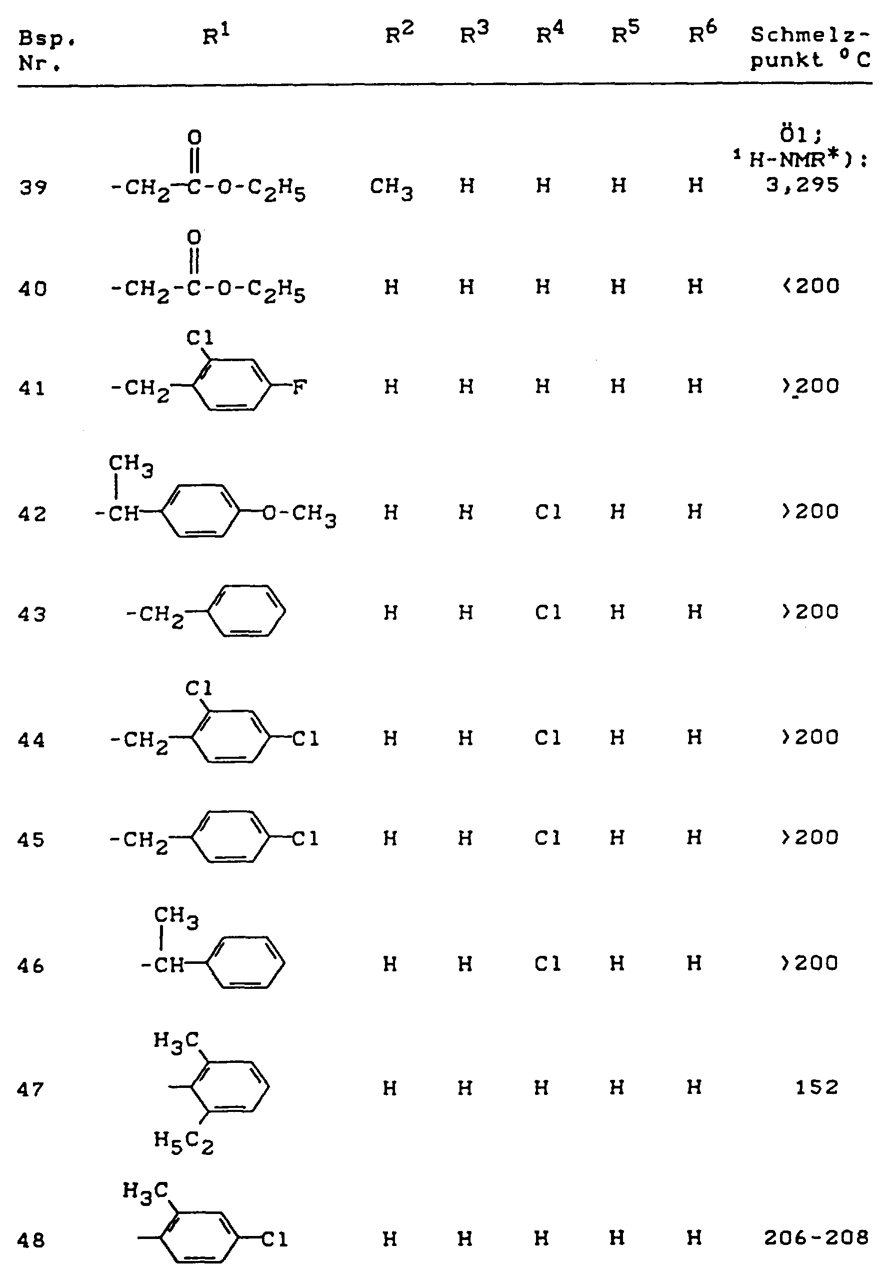

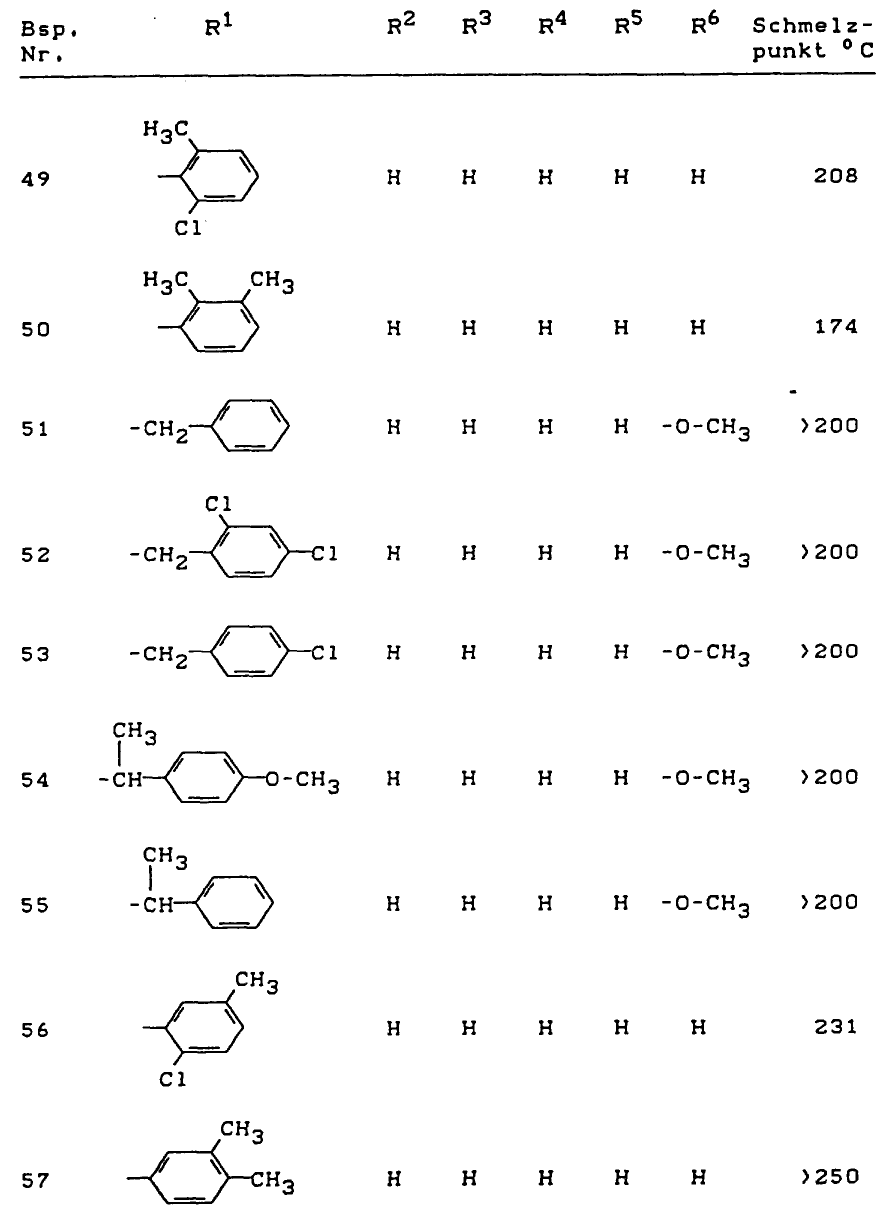

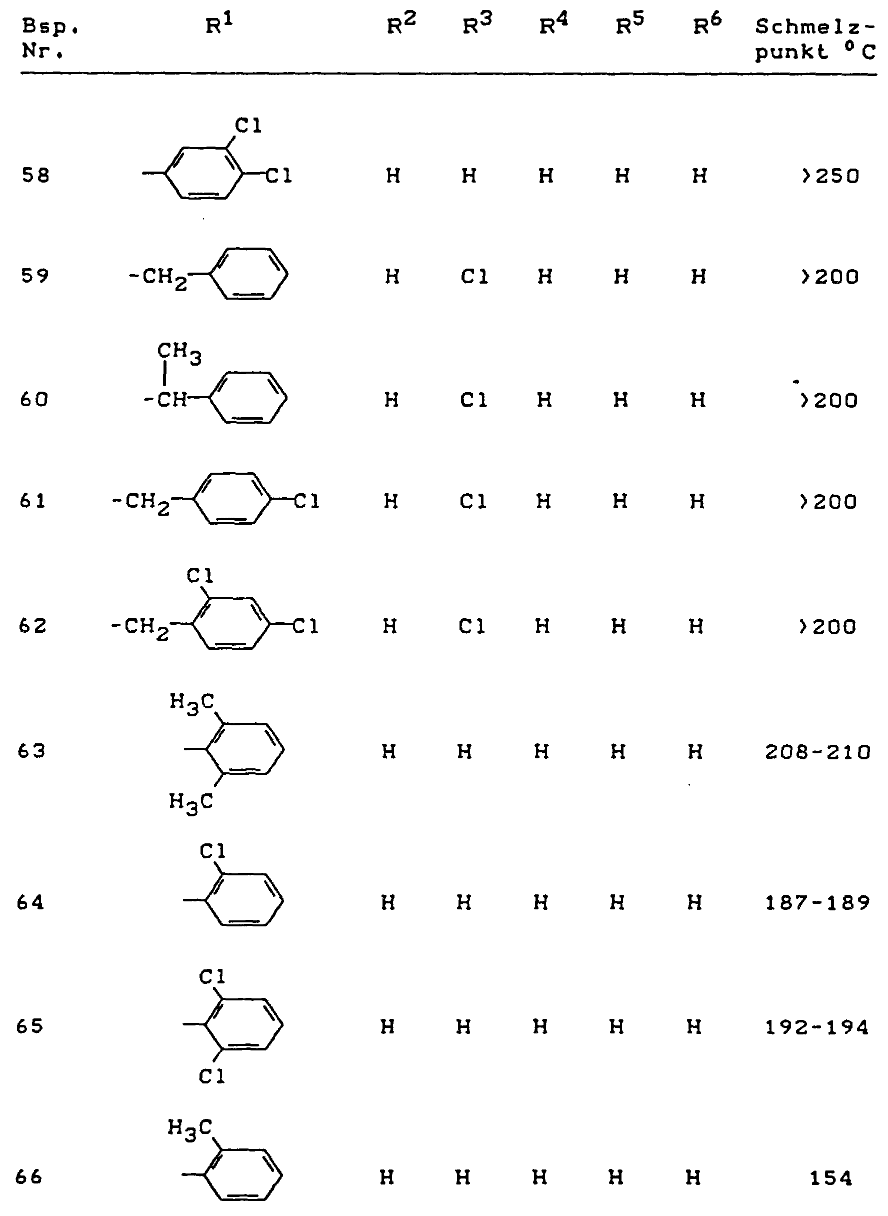

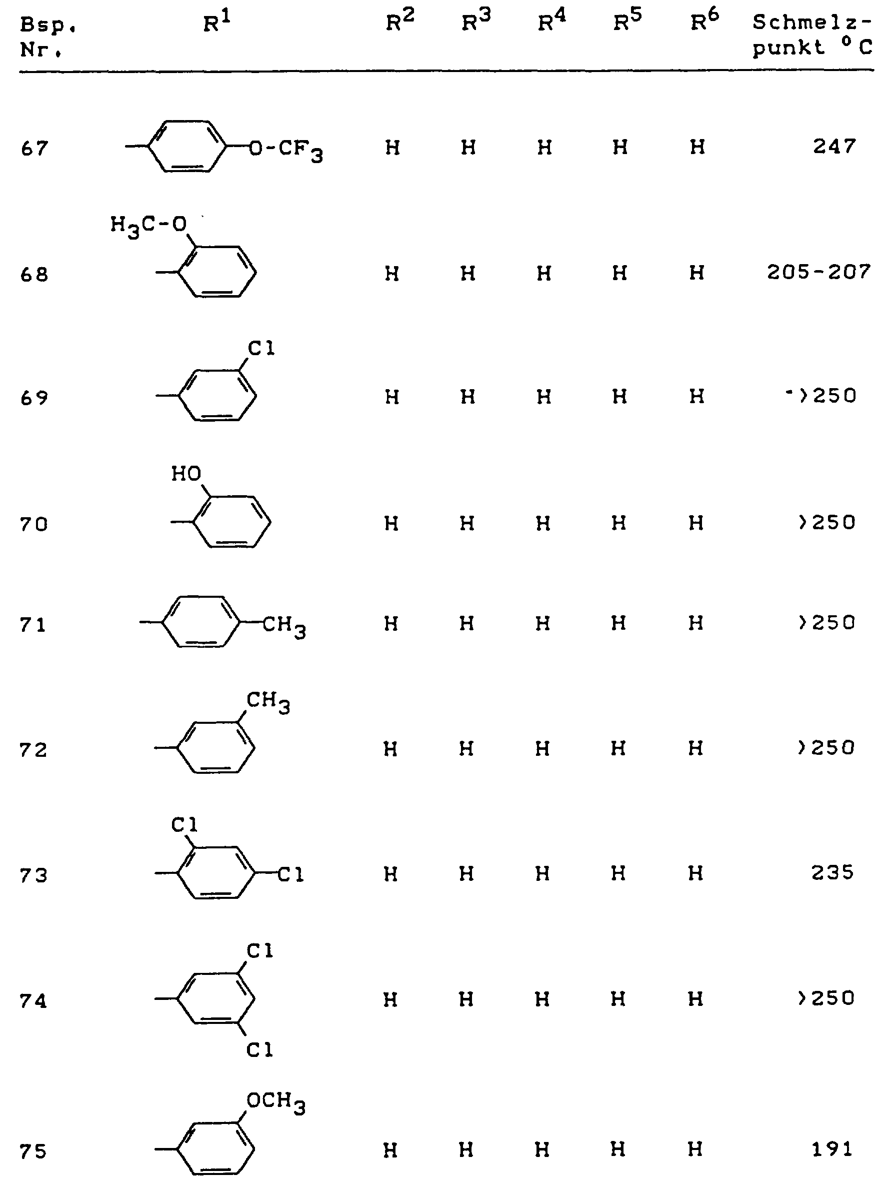

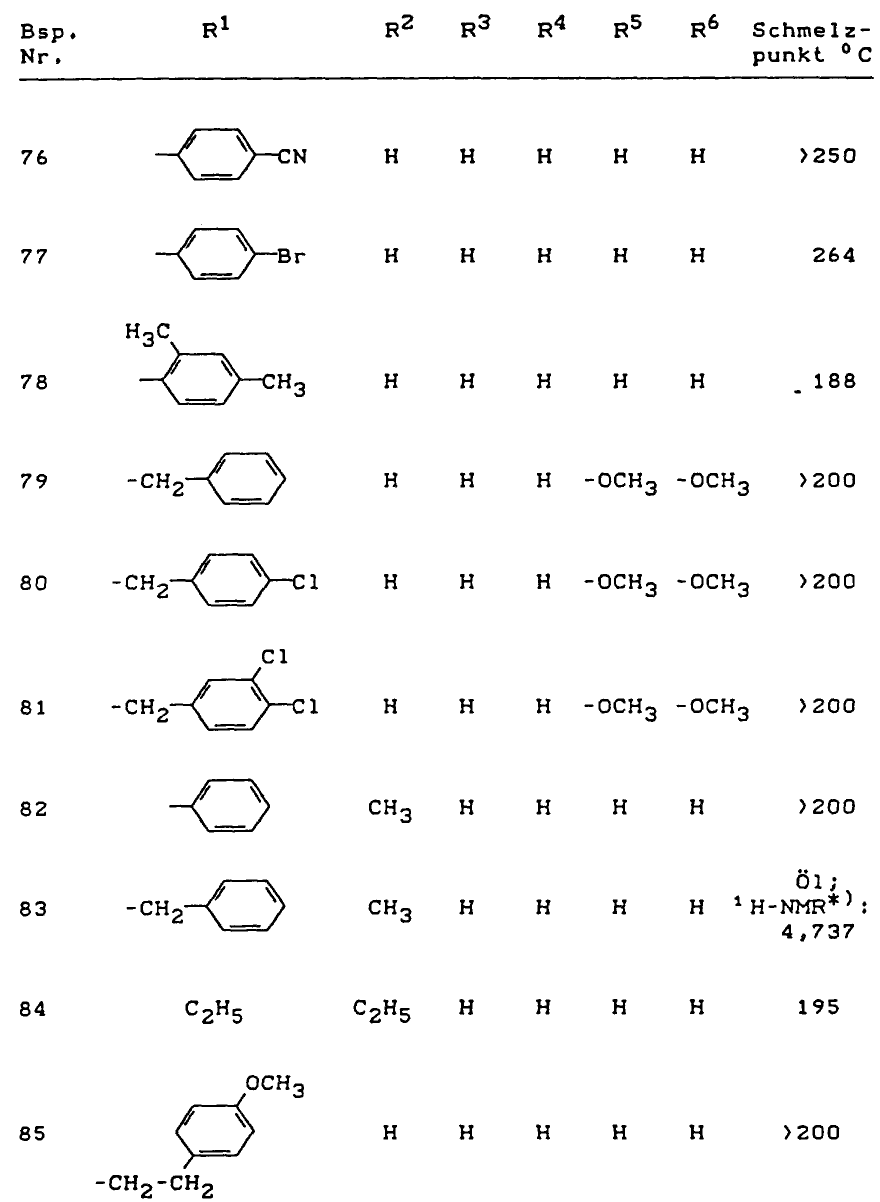

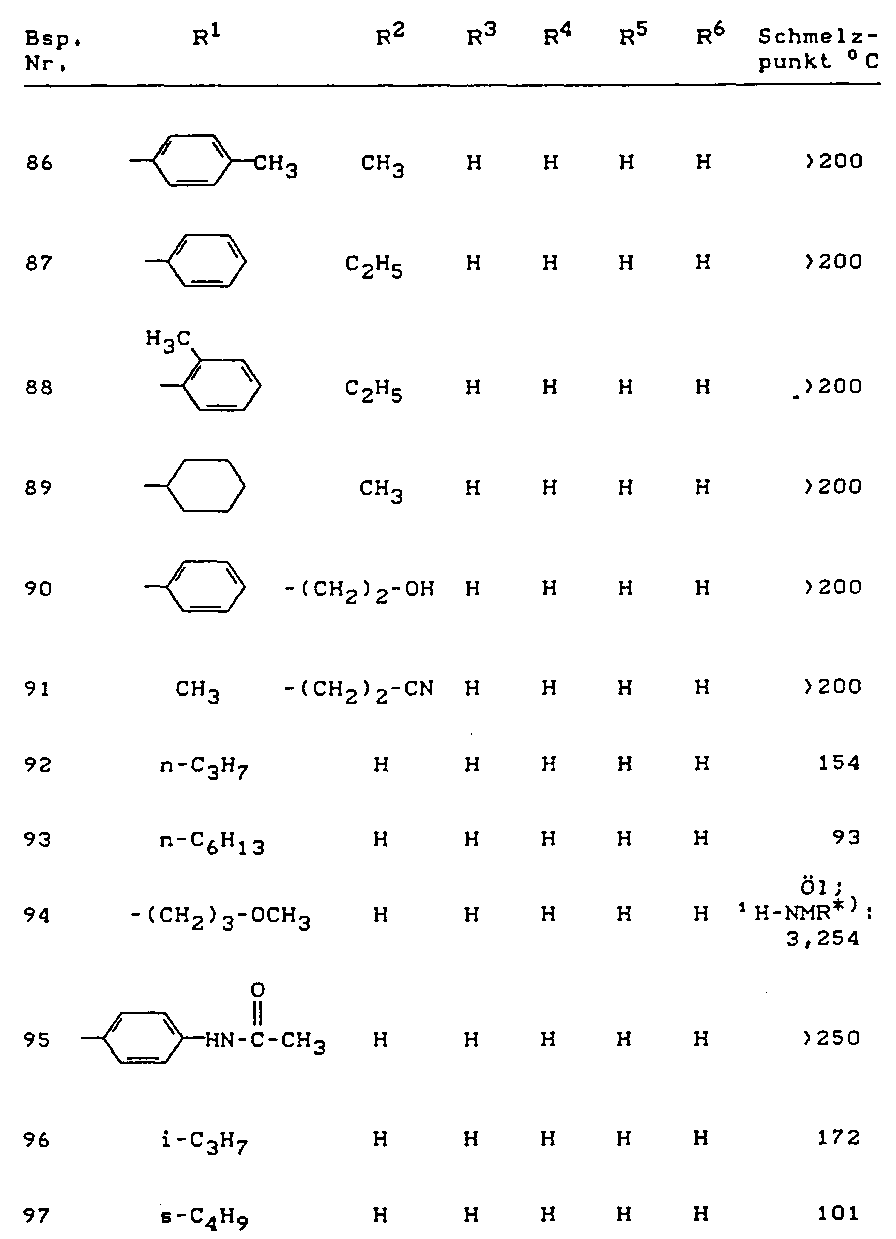

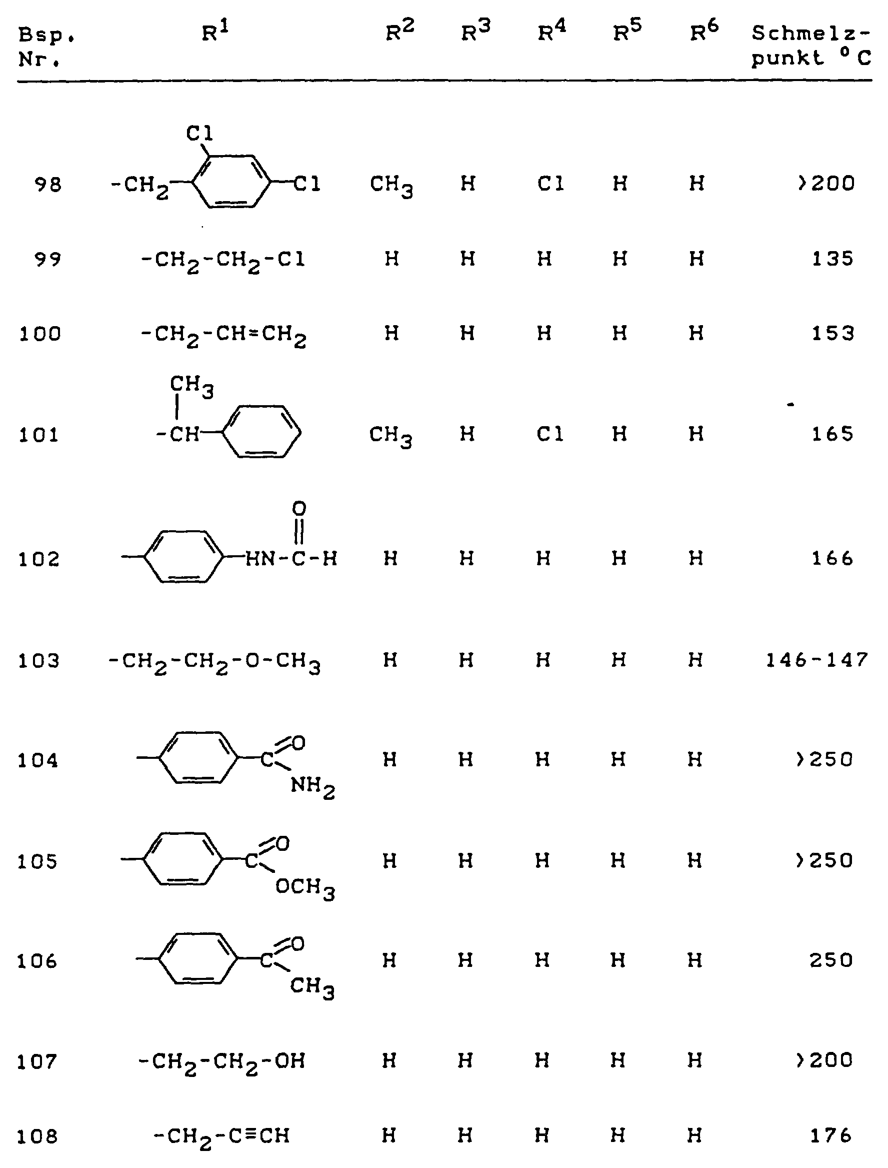

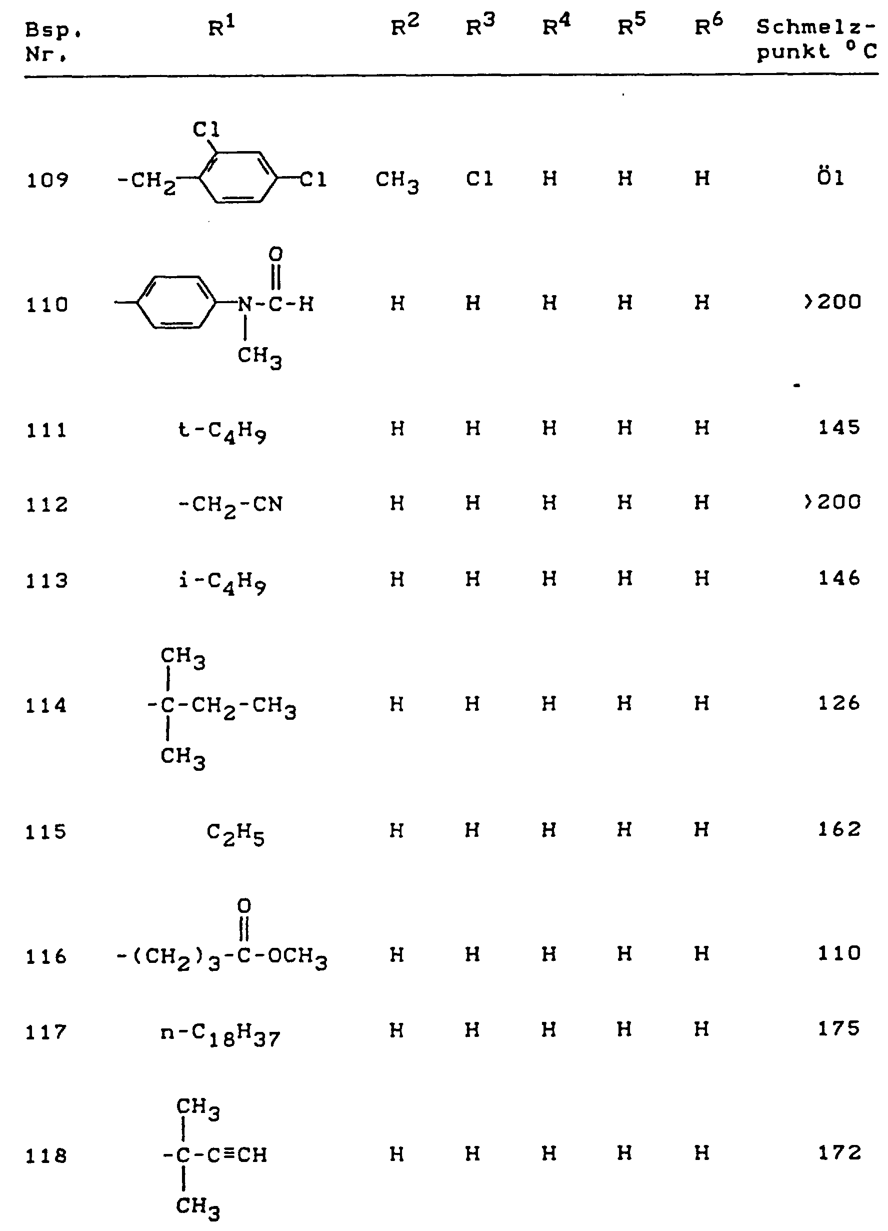

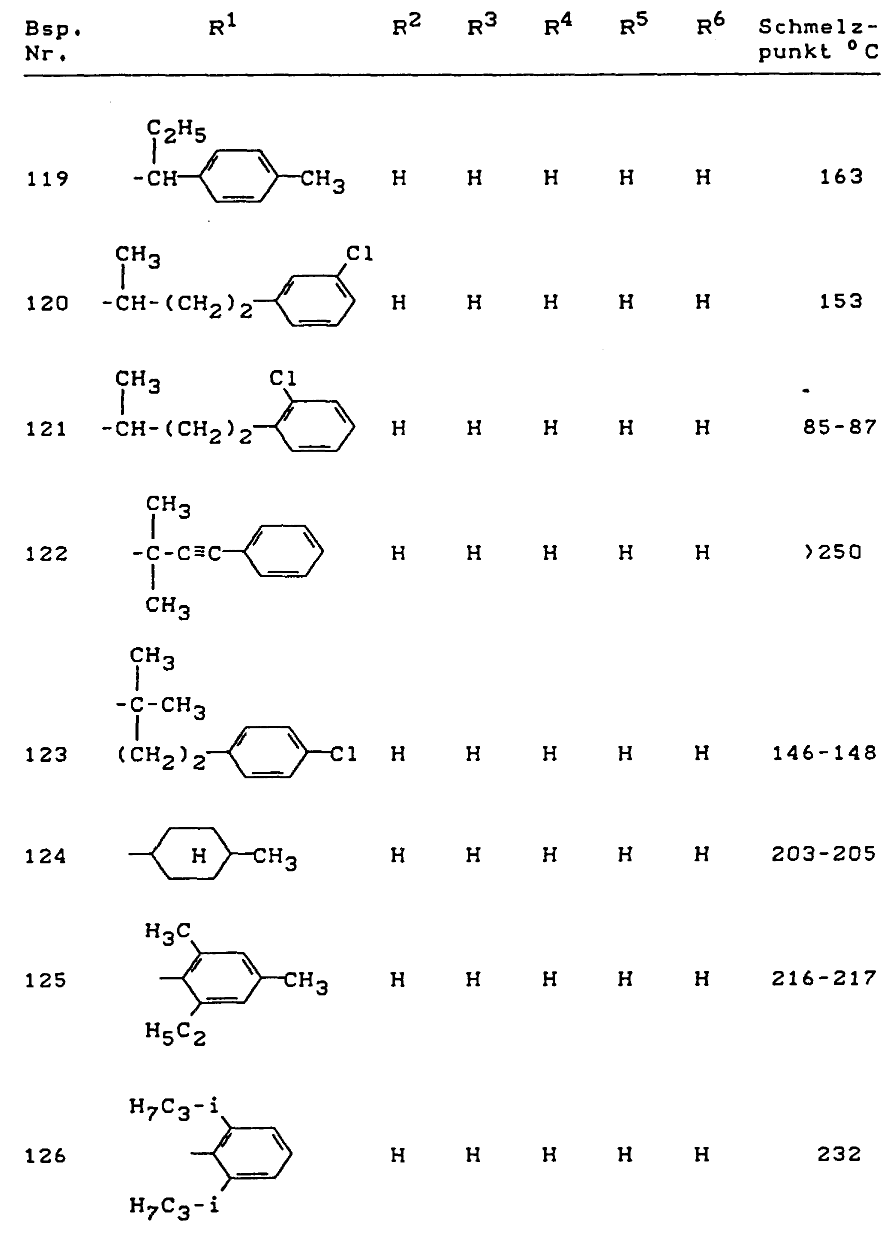

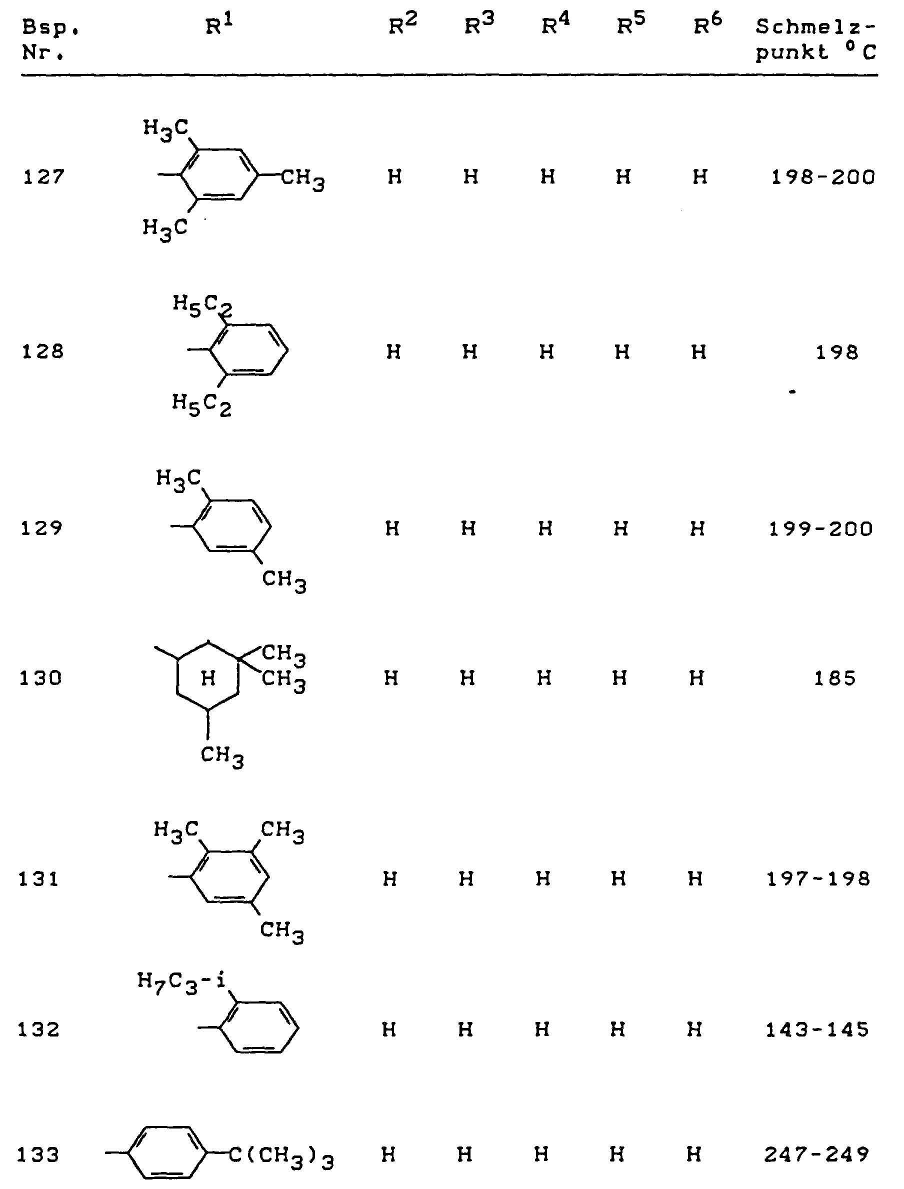

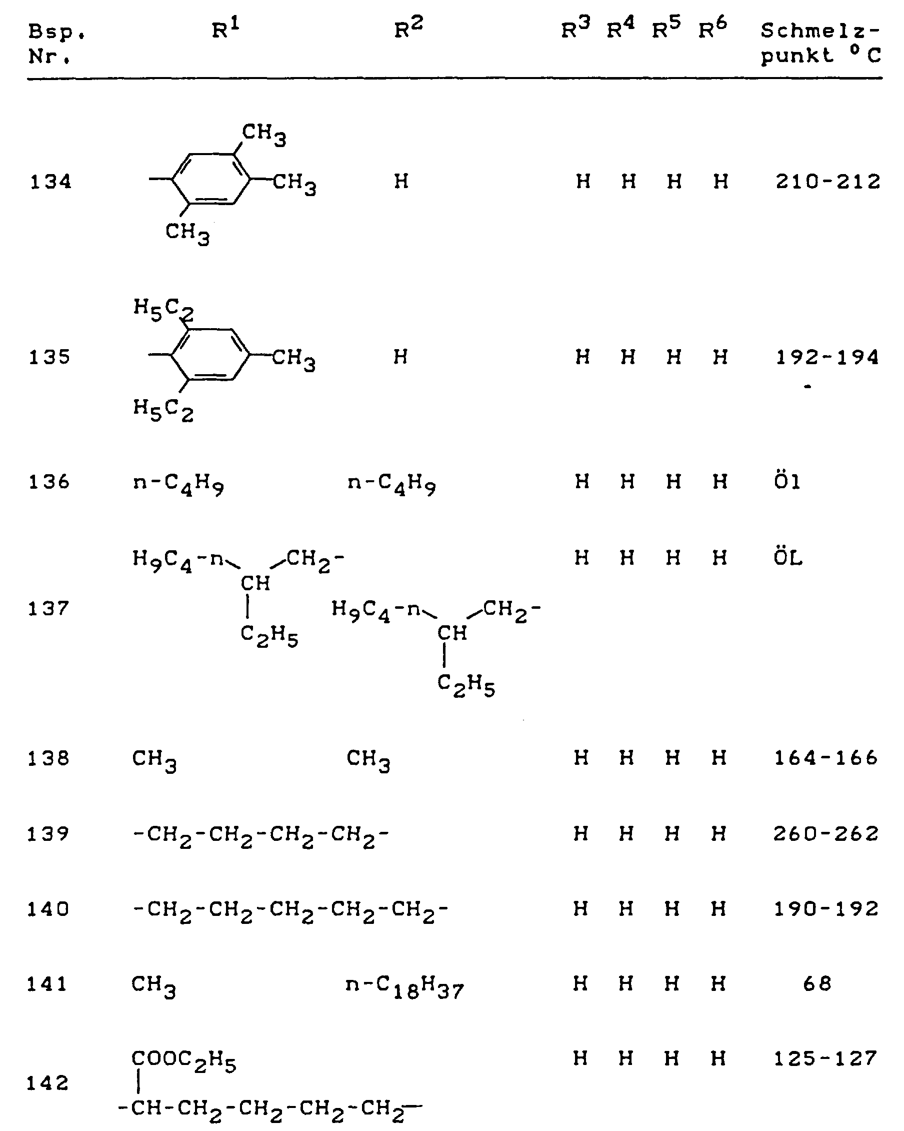

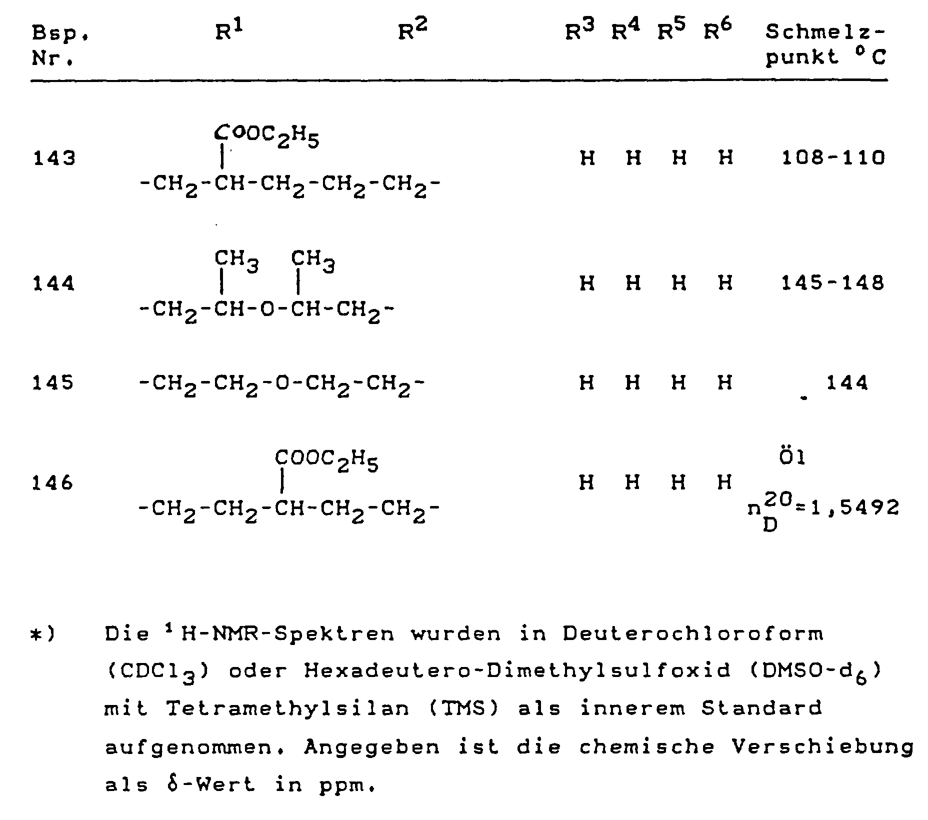

### Anwendungsbeispiele:

In den folgenden Anwendungsbeispielen A und B wurde die nachstehend aufgeführte Verbindung als Vergleichssubstanz eingesetzt: 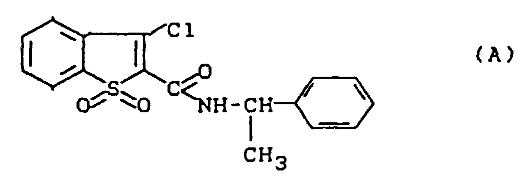 3-Chlor-N-(1-phenylethyl)-benzothiophen-2-carboxamid-S,S-dioxid (bekannt aus DE-A 38 32 848).

### Beispiel A

### Venturia-Test (Apfel) / protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen: 1, 6, 16, 17, 23, 25, 29, 30.

### Beispiel B

Leptosphaeria nodorum-Test (Weizen) / protektiv

Lösungsmittel: 100 Gewichtsteile Dimethylformamid Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutlich Überlegenheit in der Wirksamkeit gegenüber dem Stand der Technik zeigen bei diesem Test z. B. die Verbindungen gemäß den Herstellungsbeispielen: 10, 28, 35, 36, 37.

### Beispiel C

Zum Nachweis der Wirksamkeit gegen Pilze bzw. Bakterien wurden die minimalen Hemm-Konzentrationen (MHK) erfindungsgemäßer Benzothiophen-2-carboxamid-S,S-dioxide bestimmt:

Ein Agar, der aus Bierwürze und Pepton (Pilze) bzw. Fleichextrakt und Pepton (Bakterien) hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2wöchiger Lagerung bei 28°C und 60 bis 70 % rel. Luftfeuchtigkeit, wird die MHK bestimmt. MHK ist die niedrigste Konzentration an Wirkstoff, bei der keinerlei Bewuchs durch die verwendete Mikrobenart erfolgt; es wurden z.B. die Herstellungsbeispiele 93, 114, 113, 111, 92, 31, 41, 35 und 6 getestet.

Als Vergleichssubstanz wurde eingesetzt: 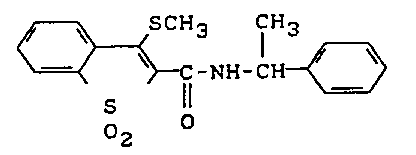 bekannt aus DE-A 3 832 848

## Patentansprüche

1. Benzothiophen-2-carboxamid-S,S-dioxide der Formel 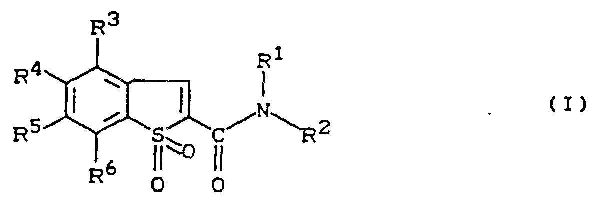 in welcher
R¹ für geradkettiges oder verzweigtes Alkyl mit 1 bis 20 Kohlenstoffatomen, für jeweils geradkettiges oder verzweigtes Halogenalkyl, Cyanalkyl, Hydroxyalkyl, Alkoxyalkyl oder Alkoxycarbonylalkyl mit jeweils 1 bis 8 Kohlenstoffatomen in den einzelnen Alkylteilen, für geradkettiges oder verzweigtes Alkenyl mit 2 bis 12 Kohlenstoffatomen, für geradkettiges oder verzweigtes Alkinyl mit 2 bis 12 Kohlenstoffatomen oder für jeweils gegebenenfalls im Cycloalkylteil ein- bis sechsfach, gleich oder verschieden substituiertes Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht, wobei als Cycloalkylsubstituenten jeweils infrage kommen: Halogen, jeweils geradkettiges oder verzweigtes Alkyl mit 1 bis 4 Kohlenstoffatomen oder jeweils geradkettiges oder verzweigtes Halogenalkyl mit 1 bis 4 Kohlenstoffatomen und 1 bis 9 gleichen oder verschiedenen Halogenatomen; außerdem für jeweils gegebenenfalls im Arylteil ein- bis fünffach, gleich oder verschieden substituiertes Arylalkyl, Arylalkenyl, Arylalkinyl oder Aryl mit jeweils 6 oder 10 Kohlenstoffatomen im Arylteil und gegebenenfalls bis zu 12 Kohlenstoffatomen im jeweils geradkettigen oder verzweigten Alkyl- bzw. Alkenyl- oder Alkinylteil steht, wobei als Arylsubstituenten jeweils infrage kommen: Halogen, Cyano, Nitro, Formylamido, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkylcarbonyl, Alkoxycarbonyl, Aminocarbonyl, N-Alkylaminocarbonyl, N,N-Dialkylaminocarbonyl, Alkylcarbonylamino, N-Alkyl-Alkylcarbonylamino, N-Alkyl-Formylcarbonylamino oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,
R für Wasserstoff oder für gegebenenfalls einfach oder mehrfach, gleich oder verschieden substituiertes, geradkettiges oder verzweigtes Alkyl mit 1 bis 6 Kohlenstoffatomen steht, wobei als Substituenten infrage kommen: Hydroxy, Halogen, Cyano sowie jeweils geradkettiges oder verzweigtes Alkoxy, Alkoxycarbonyl oder Dialkylamino mit jeweils 1 bis 6 Kohlenstoffatomen in den einzelnen Alkylteilen oder
R¹ und R gemeinsam mit dem Stickstoffatom, an welches sie gebunden sind, für einen gegebenenfalls einfach oder mehrfach, gleich oder verschieden substituierten, gesättigten 5- bis 7-gliedrigen Heterocyclus stehen, der gegebenenfalls 1 oder 2 weitere Heteroatome, insbesondere Stickstoff, Sauerstoff und/oder Schwefel, enthalten kann, wobei als Substituenten infrage kommen: Halogen, jeweils geradkettiges oder verzweigtes Alkyl, Alkoxy oder Alkoxycarbonyl mit jeweils 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen und
R³, R⁴, R⁵ und R⁶ unabhängig voneinander jeweils für Wasserstoff, Halogen, Cyano, Nitro oder für jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 13 gleichen oder verschiedenen Halogenatomen stehen.

2. Benzothiophen-2-carboxamid-S,S-dioxide der Formel (I), nach Anspruch 1, bei welchen
R¹ für geradkettiges oder verzweigtes Alkyl mit 1 bis 18 Kohlenstoffatomen, für jeweils geradkettiges oder verzweigtes Halogenalkyl, Cyanalkyl, Hydroxyalkyl, Alkoxyalkyl oder Alkoxycarbonylalkyl mit jeweils 1 bis 4 Kohlenstoffatomen in den einzelnen Alkylteilen, für geradkettiges oder verzweigtes Alkenyl mit 2 bis 8 Kohlenstoffatomen, für geradkettiges oder verzweigtes Alkinyl mit 2 bis 8 Kohlenstoffatomen oder für jeweils gegebenenfalls im Cycloalkylteil ein- bis vierfach, gleich oder verschieden substituiertes Cycloalkylalkyl oder Cycloalkyl mit jeweils 3 bis 6 Kohlenstoffatomen im Cycloalkylteil und gegebenenfalls 1 bis 4 Kohlenstoffatomen im geradkettigen oder verzweigten Alkylteil steht, wobei als Cycloalkylsubstituenten jeweils infrage kommen:
Fluor, Chlor, Brom, Methyl, Ethyl, n- oder i-Propyl, n-, i-, s- oder t-Butyl, Chlormethyl, Dichlormethyl oder Trifluormethyl; außerdem für jeweils gegebenenfalls im Arylteil ein-bis fünffach, gleich oder verschieden substituiertes Arylalkyl, Arylalkenyl, Arylalkinyl oder Aryl mit jeweils 6 oder 10 Kohlenstoffatomen im Arylteil und gegebenenfalls bis zu 8 Kohlenstoffatomen im jeweils geradkettigen oder verzweigten Alkyl- bzw. Alkenyl- oder Alkinylteil steht, wobei als Arylsubstituenten jeweils infrage kommen:
Halogen, Hydroxy, Cyano, Nitro, Formylamido, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkylcarbonyl, Alkoxycarbonyl, Aminocarbonyl, N-Alkylaminocarbonyl, N,N-Dialkylaminocarbonyl, Alkylcarbonylamino, N-Alkyl-Alkylcarbonylamino, N-Alkyl-Formylcarbonylamino oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,
R für Wasserstoff oder für gegebenenfalls einfach oder mehrfach, gleich oder verschieden substituiertes, geradkettiges oder verzweigtes Alkyl mit 1 bis 4 Kohlenstoffatomen steht, wobei als Substituenten infrage kommen: Hydroxy, Halogen, Cyano sowie jeweils geradkettiges oder verzweigtes Alkoxy, Alkoxycarbonyl oder Dialkylamino mit jeweils 1 bis 4 Kohlenstoffatomen in den einzelnen Alkylteilen oder
R¹ und R gemeinsam mit dem Stickstoffatom, an welches sie gebunden sind, für einen gegebenenfalls ein-bis vierfach, gleich oder verschieden substituierten Heterocyclus der Formel 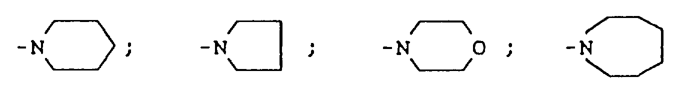 oder 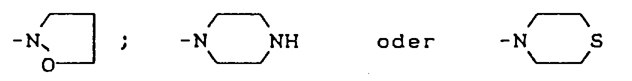 stehen, wobei als Substituenten jeweils infrage kommen: Fluor, Chlor, Brom, Methyl, Ethyl, n- oder i-Propyl, Methoxy, Ethoxy, Methoxycarbonyl, Ethoxycarbonyl, Chlormethyl, Trichlormethyl, Dichlormethyl oder Trifluormethyl und
R³, R⁴, R⁵ und R⁶ unabhängig voneinander jeweils für Wasserstoff, Halogen, Cyano, Nitro oder für jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen stehen.

3. Benzothiophen-2-carboxamid-S,S-dioxide der Formel (I) nach Anspruch 1, bei welchen
R¹ für Methyl, Ethyl, n- oder i-Propyl, n-, i-, s- oder t-Butyl, n- oder i-Pentyl, n- oder i-Hexyl, n- oder i-Heptyl, n- oder i-Octyl, n-oder i-Nonyl, n- oder i-Decyl, n- oder i-Dodecyl, n- oder i-Octadecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, Chlormethyl, Brommethyl, Chlorethyl, Bromethyl, Chlorpropyl, Brompropyl, Cyanomethyl, Cyanoethyl, Cyanopropyl, Hydroxymethyl, Hydroxyethyl, Hydroxypropyl, Methoxymethyl, Methoxyethyl, Methoxypropyl, Ethoxymethyl, Ethoxyethyl, Ethoxypropyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl; außerdem für jeweils gegebenenfalls im Cycloalkylteil ein- bis vierfach, gleich oder verschieden durch Fluor, Chlor, Brom, Methyl, Ethyl, n- oder i-Propyl, Chlormethyl, Dichlormethyl oder Trifluormethyl substituiertes Cyclopropyl, Cyclopropylmethyl, Cyclopropylethyl, Cyclopropylpropyl, Cyclopentyl, Cyclopentylmethyl, Cyclopentylethyl, Cyclopentylpropyl, Cyclohexyl, Cyclohexylmethyl, Cyclohexylethyl oder Cyclohexylpropyl steht; außerdem für jeweils gegebenenfalls im Arylteil ein- bis dreifach, gleich oder verschieden substituiertes Phenylalkyl, Phenylalkenyl, Phenylalkinyl, Phenyl oder Naphthyl mit jeweils gegebenenfalls bis zu 6 Kohlenstoffatomen im geradkettigen oder verzweigten Alkyl- bzw. Alkenyl- oder Alkinylteil steht, wobei als Arylsubstituenten jeweils infrage kommen:
Fluor, Chlor, Brom, Hydroxy, Cyano, Nitro, Methyl, Ethyl, n- oder i-Propyl, n-, i-, s-oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methylcarbonyl, Ethylcarbonyl, Methoxycarbonyl, Ethoxycarbonyl, Aminocarbonyl, N-Methylaminocarbonyl, N,N-Dimethylaminocarbonyl, N-Ethylaminocarbonyl, N,N-Diethylaminocarbonyl, N-Formylamino, N-Acetylamino, N-Methyl-N-Formylamino, N-Methyl-N-Acetyl-amino, N-Ethyl-N-Formylamino, N-Ethyl-N-Acetyl-amino, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl,
R für Wasserstoff, Methyl, Ethyl, n- oder i-Propyl, n-, i-, s- oder t-Butyl, n- oder i-Pentyl, n- oder i-Hexyl, Hydroxymethyl, Hydroxyethyl, Hydroxypropyl, Chlormethyl, Brommethyl, Chlorethyl, Bromethyl, Chlorpropyl, Brompropyl, Cyanomethyl, Cyanoethyl, Cyanopropyl, Hydroxymethyl, Hydroxyethyl, Hydroxypropyl, Methoxymethyl, Methoxyethyl, Methoxypropyl, Ethoxymethyl, Ethoxyethyl, Ethoxypropyl, Methoxycarbonylmethyl, Methoxycarbonylethyl, Methoxycarbonylpropyl, Ethoxycarbonylmethyl, Ethoxycarbonylethyl, Ethoxycarbonylpropyl, Propoxycarbonylmethyl, Propoxycarbonylethyl, Propoxycarbonylpropyl, Dimethylaminomethyl, Diethylaminomethyl, Dipropylaminomethyl, Dimethylaminoethyl, Diethylaminoethyl, Dipropylaminoethyl, Dimethylaminopropyl, Diethylaminopropyl oder Dipropylaminopropyl steht oder
R¹ und R gemeinsam mit dem Stickstoffatom, an welches sie gebunden sind, für einen gegebenenfalls einfach, zweifach oder dreifach durch Methyl und/oder Ethyl, Methoxycarbonyl und/oder Ethoxycarbonyl substituierten Heterocyclus der Formel 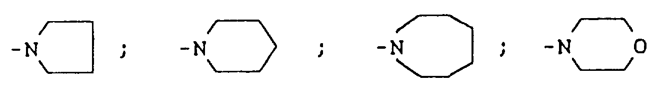 oder 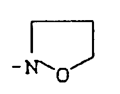 stehen und
R³, R⁴, R⁵ und R⁶ unabhängig voneinander jeweils für Wasserstoff, Fluor, Chlor, Brom, Cyano, Nitro, Methyl, Ethyl, n- oder i-Propyl, n-, i-, s-oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio stehen.

4. Verfahren zur Herstellung von Benzthiophen-2-carboxamid-S,S-dioxiden der Formel 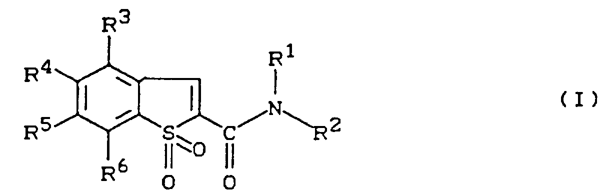 in welcher
R¹, R, R³, R⁴, R⁵ und R⁶ die im Anspruch 1 angegebenen Bedeutungen haben,
dadurch gekennzeichnet, daß man Benzothiophen-2-carboxamide der Formel 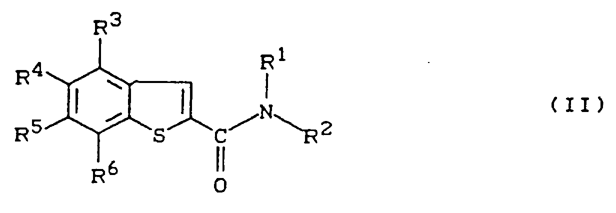 in welcher
R¹, R, R³, R⁴, R⁵ und R⁶ die oben angegebene Bedeutung haben,
mit einem Oxidationsmittel gegebenenfalls in Gegenwart eines Verdünnungsmittels und gegebenenfalls in Gegenwart eines Reaktionshilfsmittels umsetzt.

5. Schädlingsbekämpfungsmittel, gekennzeichnet durch einen Gehalt an mindestens einem Benzothiophen-2-carboxamid-S,S-dioxid der Formel (1) gemäß Anspruch 1.

6. Verwendung von Benzothiophen-2-carboxamid-S,S-dioxiden der Formel (1) gemäß Anspruch 1 zur Bekämpfung von Schädlingen auf dem Gebiet des Pflanzenschutzes und im Materialschutz.

7. Verfahren zur Bekämpfung von Schädlingen auf dem Gebiet des Pflanzenschutzes und im Materialschutz, dadurch gekennzeichnet, daß man Benzothiophen-2-carboxamid-S,S-dioxide der Formel (1) gemäß Anspruch 1 auf die Schädlinge und/oder deren Lebensraum ausbringt.

8. Verfahren zur Herstellung von Schädlingsbekämpfungmitteln, dadurch gekennzeichnet, daß man Benzothiophen-2-carboxamid-S,S-dioxide der Formel (1) gemäß Anspruch 1 mit Streckmitteln und/oder oberflächenaktiven Stoffen vermischt.

## Claims

1. Benzothiophene-2-carboxamide S,S-dioxides of the formula (I) 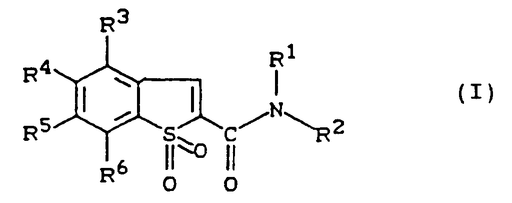 in which
R¹ represents straight-chain or branched alkyl having 1 to 20 carbon atoms, or represents in each case straight-chain or branched halogenoalkyl, cyanoalkyl, hydroxyalkyl, alkoxyalkyl or alkoxycarbonylalkyl, each of which has 1 to 8 carbon atoms in the individual alkyl moieties, or represents straight-chain or branched alkenyl having 2 to 12 carbon atoms, or represents straight-chain or branched alkinyl having 2 to 12 carbon atoms, or represents cycloalkylalkyl or cycloalkyl, each of which has 3 to 7 carbon atoms in the cycloalkyl moiety and, if appropriate, 1 to 6 carbon atoms in the straight-chain or branched alkyl moiety, and each of which is optionally monosubstituted to hexasubstituted in the cycloalkyl moiety by identical or different substituents, suitable cycloalkyl substituents in each case being: halogen, in each case straight-chain or branched alkyl having 1 to 4 carbon atoms or in each case straight-chain or branched halogenoalkyl having 1 to 4 carbon atoms and 1 to 9 identical or different halogen atoms; furthermore represents arylalkyl, arylalkenyl, arylalkinyl or aryl, each of which has 6 or 10 carbon atoms in the aryl moiety and, if appropriate, up to 12 carbon atoms in the respective straight-chain or branched alkyl or alkenyl or alkinyl moiety, each of which is optionally monosubstituted to pentasubstituted in the aryl moiety by identical or different substituents, suitable aryl substituents in each case being: halogen, cyano, nitro, formylamido, in each case straight-chain or branched alkyl, alkoxy or alkylthio, each of which has 1 to 4 carbon atoms, in each case straight-chain or branched halogenoalkyl, halogenoalkoxy, halogenoalkylthio, each of which has 1 to 4 carbon atoms and 1 to 9 identical or different halogen atoms, in each case straight-chain or branched alkylcarbonyl, alkoxycarbonyl, aminocarbonyl, N-alkylaminocarbonyl, N,N-dialkylaminocarbonyl, alkylcarbonylamino, N-alkyl-alkylcarbonylamino, N-alkyl-formylcarbonylamino or alkoximinoalkyl, each of which has 1 to 4 carbon atoms in the individual alkyl moieties, and phenyl which is optionally monosubstituted or polysubstituted by identical or different substituents from the series comprising halogen and/or straight-chain or branched alkyl having 1 to 4 carbon atoms,
R represents hydrogen or straight-chain or branched alkyl which has 1 to 6 carbon atoms and which is optionally monosubstituted or polysubstituted by identical or different substituents, suitable substituents being: hydroxyl, halogen, cyano, and in each case straight-chain or branched alkoxy, alkoxycarbonyl or dialkylamino, each of which has 1 to 6 carbon atoms in the individual alkyl moieties, or
R¹ and R together with the nitrogen atom to which they are bonded represent a saturated 5- to 7-membered heterocycle which is optionally monosubstituted or polysubstituted by identical or different substituents and which can optionally contain 1 or 2 further hetero atoms, in particular nitrogen, oxygen and/or sulphur, suitable substituents in each case being: halogen, in each case straight-chain or branched alkyl, alkoxy or alkoxycarbonyl having in each case 1 to 4 carbon atoms or straight-chain or branched halogenoalkyl having 1 to 4 carbon atoms and 1 to 9 identical or different halogen atoms and
R³, R⁴, R⁵ and R⁶ independently of one another in each
case represent hydrogen, halogen, cyano, nitro or in each case straight-chain or branched alkyl, alkoxy, alkylthio, halogenoalkyl, halogenoalkoxy or halogenoalkylthio each of which has 1 to 6 carbon atoms and if appropriate 1 to 13 identical or different halogen atoms.

2. Benzothiophene-2-carboxamide S,S-dioxides of the formula (I), according to Claim 1, in which
R¹ represents straight-chain or branched alkyl having 1 to 18 carbon atoms, in each case straight-chain or branched halogenoalkyl, cyanoalkyl, hydroxyalkyl, alkoxyalkyl or alkoxycarbonylalkyl, each of which has 1 to 4 carbon atoms in the individual alkyl moieties, or represents straight-chain or branched alkenyl having 2 to 8 carbon atoms, or represents straight-chain or branched alkinyl having 2 to 8 carbon atoms, or represents cycloalkylalkyl or cycloalkyl, each of which has 3 to 6 carbon atoms in the cycloalkyl moiety and, if appropriate, 1 to 4 carbon atoms in the straight-chain or branched alkyl moiety and each of which is optionally monosubstituted to tetrasubstituted in the cycloalkyl moiety by identical or different substituents, suitable cycloalkyl substituents in each case being:
fluorine, chlorine, bromine, methyl, ethyl, n- or i-propyl, n-, i-, s- or t-butyl, chloromethyl, dichloromethyl or trifluoromethyl; moreover arylalkyl, arylalkenyl, arylalkinyl or aryl, each of which has 6 or 10 carbon atoms in the aryl moiety and, if appropriate, up to 8 carbon atoms in the particular straight-chain or branched alkyl or alkenyl or alkinyl moiety and each of which is optionally monosubstituted to pentasubstituted in the aryl moiety by identical or different substituents, suitable aryl substituents in each case being:
halogen, hydroxyl, cyano, nitro, formylamido, in each case straight-chain or branched alkyl, alkoxy or alkylthio, each of which has 1 to 4 carbon atoms, in each case straight-chain or branched halogenoalkyl, halogenoalkoxy, halogenoalkylthio, each of which has 1 to 4 carbon atoms and 1 to 9 identical or different halogen atoms, in each case straight-chain or branched alkylcarbonyl, alkoxycarbonyl, aminocarbonyl, N-alkylaminocarbonyl, N,N-dialkylaminocarbonyl, alkylcarbonylamino, N-alkyl-alkylcarbonylamino, N-alkyl-formylcarbonylamino or alkoximinoalkyl, each of which has 1 to 4 carbon atoms in the individual alkyl moieties, and phenyl which is optionally monosubstituted or polysubstituted by identical or different substituents from the series comprising halogen and/or straight-chain or branched alkyl having 1 to 4 carbon atoms,
R represents hydrogen or alkyl which has 1 to 4 carbon atoms and which is optionally monosubstituted or polysubstituted by identical or different substituents, suitable substituents being: hydroxyl, halogen, cyano and in each case straight-chain or branched alkoxy, alkoxycarbonyl or dialkylamino, each of which has 1 to 4 carbon atoms in the individual alkyl moieties, or
R¹ and R together with the nitrogen atom to which they are bonded represent a heterocycle of the formula 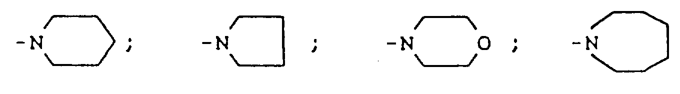 or 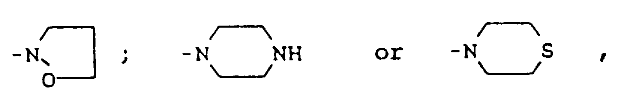 each of which is optionally monosubstituted to tetrasubstituted by identical or different substituents, suitable substituents in each case being: fluorine, chlorine, bromine, methyl, ethyl, n- or i-propyl, methoxy, ethoxy, methoxycarbonyl, ethoxycarbonyl, chloromethyl, trichloromethyl, dichloromethyl or trifluoromethyl, and
R³, R⁴, R⁵ and R⁶ independently of one another in each
case represent hydrogen, halogen, cyano, nitro or in each case straight-chain or branched alkyl, alkoxy, alkylthio, halogenoalkyl, halogenoalkoxy or halogenoalkylthio, each of which has 1 to 4 carbon atoms and, if appropriate, 1 to 9 identical or different halogen atoms.

3. Benzothiophene-2-carboxamide S,S-dioxides of the formula (I) according to Claim 1, in which
R¹ represents methyl, ethyl, n- or i-propyl, n-, i-, s- or t-butyl, n- or i-pentyl, n- or i-hexyl, nor i-heptyl, n- or i-octyl, n- or i-nonyl, n- or i-decyl, n- or i-dodecyl, n- or i-octadecyl, allyl, n- or i-butenyl, n- or i-pentenyl, n- or i-hexenyl, propargyl, n- or i-butinyl, n- or i-pentinyl, n- or i-hexinyl, chloromethyl, bromomethyl, chloroethyl, bromoethyl, chloropropyl, bromopropyl, cyanomethyl, cyanoethyl, cyanopropyl, hydroxymethyl, hydroxyethyl, hydroxypropyl, methoxymethyl, methoxyethyl, methoxypropyl, ethoxymethyl, ethoxyethyl, ethoxypropyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl; furthermore represents cyclopropyl, cyclopropylmethyl, cyclopropylethyl, cyclopropylpropyl, cyclopentyl, cyclopentylmethyl, cyclopentylethyl, cyclopentylpropyl, cyclohexyl, cyclohexylmethyl, cyclohexylethyl or cyclohexylpropyl, each of which is optionally monosubstituted to tetrasubstituted in the cycloalkyl moiety by identical or different substituents from the series comprising fluorine, chlorine, bromine, methyl, ethyl, n- or i-propyl, chloromethyl, dichloromethyl or trifluoromethyl; furthermore represents phenylalkyl, phenylalkenyl, phenylalkinyl, phenyl or naphthyl, each of which has, if appropriate, up to 6 carbon atoms in the straight-chain or branched alkyl or alkenyl or alkinyl moiety and each of which is optionally monosubstituted to trisubstituted in the aryl moiety by identical or different substituents, suitable aryl substituents in each case being:
fluorine, chlorine, bromine, hydroxyl, cyano, nitro, methyl, ethyl, n- or i-propyl, n-, i-, s-or t-butyl, methoxy, ethoxy, n- or i-propoxy, n-, i-, s- or t-butoxy, methylthio, ethylthio, trifluoromethyl, trifluoromethoxy, trifluoromethylthio, methylcarbonyl, ethylcarbonyl, methoxycarbonyl, ethoxycarbonyl, aminocarbonyl, N-methylaminocarbonyl, N,N-dimethylaminocarbonyl, N-ethylaminocarbonyl, N,N-diethylaminocarbonyl, N-formylamino, N-acetylamino, N-methyl-N-formylamino, N-methyl-N-acetylamino, N-ethyl-N-formylamino, N-ethyl-N-acetylamino, methoximinomethyl, methoximinoethyl, ethoximinomethyl, ethoximinoethyl, or phenyl which is optionally monosubstituted to trisubstituted by identical or different substituents from the series comprising fluorine, chlorine, bromine, methyl and/or ethyl,
R represents hydrogen, methyl, ethyl, n- or i-propyl, n-, i-, s- or t-butyl, n- or i-pentyl, n- or i-hexyl, hydroxymethyl, hydroxyethyl, hydroxypropyl, chloromethyl, bromomethyl, chloroethyl, bromoethyl, chloropropyl, bromopropyl, cyanomethyl, cyanoethyl, cyanopropyl, hydroxymethyl, hydroxyethyl, hydroxypropyl, methoxymethyl, methoxyethyl, methoxypropyl, ethoxymethyl, ethoxyethyl, ethoxypropyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl, dimethylaminomethyl, diethylaminomethyl, dipropylaminomethyl, dimethylaminoethyl, diethylaminoethyl, dipropylaminoethyl, dimethylaminopropyl, diethylaminopropyl or dipropylaminopropyl or
R¹ and R together with the nitrogen atom to which they are bonded represent a heterocycle of the formula 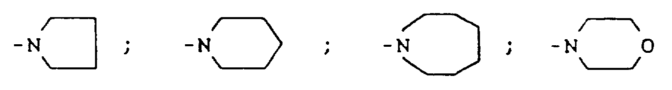 or 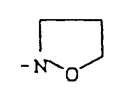
each of which is optionally monosubstituted, disubstituted or trisubstituted by methyl and/or ethyl, methoxycarbonyl and/or ethoxycarbonyl and
R³, R⁴, R⁵ and R⁶ independently of one another in each
case represent hydrogen, fluorine, chlorine, bromine, cyano, nitro, methyl, ethyl, n- or i-propyl, n-, i-, s- or t-butyl, methoxy, ethoxy n- or i-propoxy, n-, i-, s- or t-butoxy, methylthio, ethylthio, trifluoromethyl, trifluoromethoxy or trifluoromethylthio.

4. Process for the preparation of benzothiophene-2-carboxamide S,S-dioxides of the formula 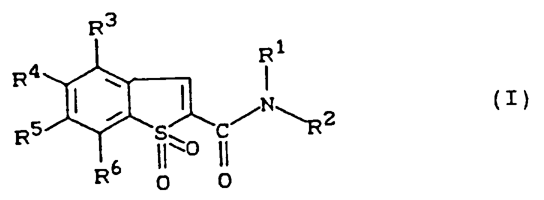
in which
R¹, R, R³, R⁴, R⁵ and R⁶ have the meanings specified in Claim 1,
characterised in that benzothiophene-2-carboxamides of the formula 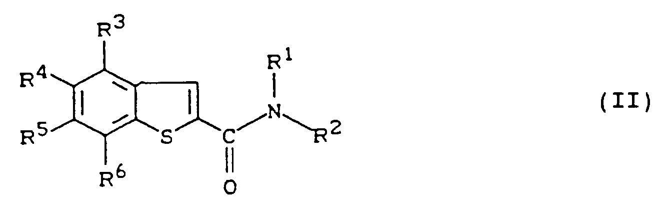 in which
R¹, R, R³, R⁴, R⁵ and R⁶ have the abovementioned meaning,
are reacted with an oxidant, if appropriate in the presence of a diluent and, if appropriate, in the presence of a reaction auxiliary.

5. Pesticide, characterised by a content of at least one benzothiophene-2-carboxamide S,S-dioxide of the formula (I) according to Claim 1.

6. Use of benzothiophene-2-carboxamide S,S-dioxides of the formula (I) according to Claim 1 for combating pests in the field of plant protection and in the protection of materials.

7. Method for combating pests in the field of plant protection and in the protection of materials, characterised in that benzothiophene-2-carboxamide S,S-dioxides of the formula (I) according to Claim 1 are applied to pests and/or on their environment.

8. Method of preparing pesticides, characterised in that benzothiophene-2-carboxamide S,S-dioxides of the formula (I) according to Claim 1 are mixed with extenders and/or surface-active substances.

## Revendications

1. S,S-dioxydes de benzothiophène-2-carboxamides de formule 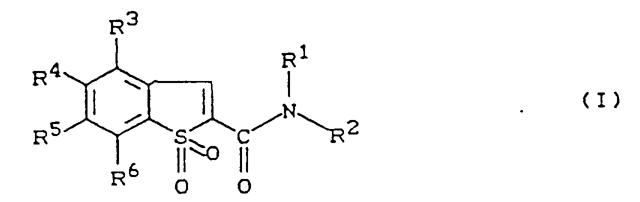 dans laquelle
R¹ est un groupe alkyle linéaire ou ramifié ayant 1 à 20 atomes de carbone, un groupe halogénalkyle, cyanalkyle, hydroxyalkyle, alkoxyalkyle ou alkoxycarbonylalkyle linéaire ou ramifié ayant dans chaque cas 1 à 8 atomes de carbone dans les parties alkyle individuelles, un groupe alcényle linéaire ou ramifié ayant 2 à 12 atomes de carbone, un groupe alcynyle linéaire ou ramifié ayant 2 à 12 atomes de carbone ou un groupe cycloalkylalkyle ou cycloalkyle portant chacun le cas échéant dans la partie cycloalkyle un à six substituants identiques ou différents et ayant chacun 3 à 7 atomes de carbone dans la partie cycloalkyle et le cas échéant 1 à 6 atomes de carbone dans la partie alkyle linéaire ou ramifiée, en considérant dans chaque cas comme substituants de la partie cycloalkyle : un halogène, un reste alkyle linéaire ou ramifié ayant 1 à 4 atomes de carbone ou un reste halogénalkyle linéaire ou ramifié ayant 1 à 4 atomes de carbone et 1 à 9 atomes d'halogènes identiques ou différents ; en outre un groupe arylalkyle, arylalcényle, arylalcynyle ou aryle portant éventuellement dans chaque cas un à cinq substituants identiques ou différents dans la partie aryle et ayant chacun 6 ou 10 atomes de carbone dans la partie aryle et le cas échéant jusqu'à 12 atomes de carbone dans la partie alkyle ou alcényle ou alcynyle à chaîne droite ou ramifiée, en considérant comme substituants de la partie aryle : un halogène, un reste cyano, nitro, formylamido, un reste alkyle, alkoxy ou alkylthio linéaire ou ramifié ayant chacun 1 à 4 atomes de carbone, un reste halogénalkyle, halogénalkoxy, halogénalkylthio linéaire ou ramifié ayant chacun 1 à 4 atomes de carbone et 1 à 9 atomes d'halogènes identiques ou différents, un reste alkylcarbonyle, alkoxycarbonyle, aminocarbonyle, N-alkylaminocarbonyle, N,N-dialkylaminocarbonyle, alkylcarbonylamino, N-alkyl-alkylcarbonylamino, N-alkyl-formylcarbonylamino ou alkoximinoalkyle linéaire ou ramifié ayant chacun 1 à 4 atomes de carbone dans les parties alkyle individuelles, ainsi qu'un reste phényle portant le cas échéant un ou plusieurs substituants, identiques ou différents, halogéno et/ou alkyle linéaires ou ramifiés ayant 1 à 4 atomes de carbone,
R est de l'hydrogène ou un groupe alkyle linéaire ou ramifié ayant 1 à 6 atomes de carbone, portant le cas échéant un ou plusieurs substituants identiques ou différents, en considérant comme substituants : un radical hydroxy, halogéno, cyano ainsi qu'alkoxy, alkoxycarbonyle ou dialkylamino linéaire ou ramifié ayant dans chaque cas 1 à 6 atomes de carbone dans les parties alkyle individuelles, ou bien
R¹ et R forment, conjointement avec l'atome d'azote auquel ils sont liés, un hétérocycle pentagonal à heptagonal saturé portant le cas échéant un ou plusieurs substituants identiques ou différents, qui peut contenir éventuellement 1 ou 2 autres hétéroatomes, notamment d'azote, d'oxygène et/ou de soufre, en considérant comme substituants : un halogène, un radical alkyle, alkoxy ou alkoxycarbonyle linéaire ou ramifié ayant chacun 1 à 4 atomes de carbone ou un radical halogénalkyle linéaire ou ramifié ayant 1 à 4 atomes de carbone et 1 à 9 atomes d'halogènes identiques ou différents et
R³, R⁴, R⁵ et R⁶ représentent chacun, indépendamment les uns des autres, de l'hydrogène, un halogène, un groupe cyano, nitro ou un groupe alkyle, alkoxy, alkylthio, halogénalkyle, halogénalkoxy ou halogénalkylthio linéaire ou ramifié ayant chacun 1 à 6 atomes de carbone et, le cas échéant, 1 à 13 atomes d'halogènes identiques ou différents.

2. S,S-dioxydes de benzothiophène-2-carboxamides de formule (I) suivant la revendication 1, dans lesquels :
R¹ est un groupe alkyle linéaire ou ramifié ayant 1 à 18 atomes de carbone, un groupe halogénalkyle, cyanalkyle, hydroxyalkyle, alkoxyalkyle ou alkoxycarbonylalkyle linéaire ou ramifié ayant chacun 1 à 4 atomes de carbone dans les parties alkyle individuelles, un groupe alcényle linéaire ou ramifié ayant 2 à 8 atomes de carbone, un groupe alcynyle linéaire ou ramifié ayant 2 à 8 atomes de carbone ou un groupe cycloalkylalkyle ou cycloalkyle portant le cas échéant un à quatre substituants identiques ou différents dans la partie cycloalkyle, ayant chacun 3 à 6 atomes de carbone dans la partie cycloalkyle et le cas échéant 1 à 4 atomes de carbone dans la partie alkyle linéaire ou ramifiée, en considérant comme substituants de la partie cycloalkyle :
du fluor, du chlore, du brome, un radical méthyle, éthyle, n-propyle, isopropyle, n-butyle, isobutyle, sec.-butyle, tertio-butyle, chlorométhyle, dichlorométhyle ou trifluorométhyle ; en outre, un groupe arylalkyle, arylalcényle, arylalcynyle ou aryle portant le cas échéant dans la partie aryle un à cinq substituants identiques ou différents et ayant chacun 6 ou 10 atomes de carbone dans la partie aryle et, le cas échéant, jusqu'à 8 atomes de carbone dans la partie alkyle ou alcényle ou alcynyle linéaire ou ramifiée, en considérant comme substituants de la partie aryle :
un halogène, un radical hydroxy, cyano, nitro, formylamido, alkyle, alkoxy ou alkylthio linéaire ou ramifié ayant chacun 1 à 4 atomes de carbone, halogénalkyle, halogénalkoxy, halogénalkylthio linéaire ou ramifié ayant chacun 1 à 4 atomes de carbone et 1 à 9 atomes d'halogènes identiques ou différents, alkylcarbonyle, alkoxycarbonyle, aminocarbonyle, N-alkylaminocarbonyle, N,N-dialkylaminocarbonyle, alkylcarbonylamino, N-alkylalkylcarbonylamino, N-alkylformylcarbonylamino ou alkoximinoalkyle linéaire ou ramifié ayant chacun 1 à 4 atomes de carbone dans les parties alkyle individuelles, ainsi qu'un groupe phényle portant le cas échéant un ou plusieurs substituants, identiques ou différents, halogéno et/ou alkyle linéaire ou ramifié ayant 1 à 4 atomes de carbone,
R est de l'hydrogène ou un groupe alkyle linéaire ou ramifié ayant 1 à 4 atomes de carbone, portant le cas échéant un ou plusieurs substituants identiques ou différents, en considérant comme substituants : un radical hydroxy, halogéno, cyano, ainsi qu'un radical alkoxy, alkoxycarbonyle ou dialkylamino linéaire ou ramifié ayant dans chaque cas 1 à 4 atomes de carbone dans les parties alkyle individuelles, ou bien
R¹ et R forment conjointement avec l'atome d'azote auquel ils sont liés un hétérocycle de formule 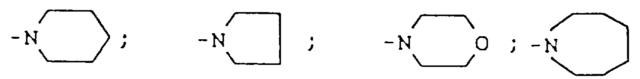 ou 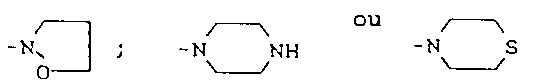 portant le cas échéant un à quatre substituants identiques ou différents, en considérant dans chaque cas comme substituants : du fluor, du chlore, du brome, un radical méthyle, éthyle, n-propyle, isopropyle, méthoxy, éthoxy, méthoxycarbonyle, éthoxycarbonyle, chlorométhyle, trichlorométhyle, dichlorométhyle ou trifluorométhyle et
R³, R⁴, R⁵ et R⁶ représentent chacun, indépendamment les uns des autres, de l'hydrogène, un halogène, un groupe cyano, nitro, ou un groupe alkyle, alkoxy, alkylthio, halogénalkyle, halogénalkoxy ou halogénalkylthio linéaire ou ramifié ayant chacun 1 à 4 atomes de carbone et, le cas échéant, 1 à 9 atomes d'halogènes identiques ou différents.

3. S,S-dioxydes de benzothiophène-2-carboxamides de formule (I) suivant la revendication 1, dans lesquels
R¹ est un groupe méthyle, éthyle, n-propyle, isopropyle, n-butyle, isobutyle, sec.-butyle, tertiobutyle, n-pentyle, isopentyle, n-hexyle, isohexyle, n-heptyle, isoheptyle, n-octyle, isooctyle, n-nonyle, isononyle, n-décyle, isodécyle, n-dodécyle, isododécyle, n-octadécyle, isooctadécyle, allyle, n-butényle, isobutényle, n-pentényle, isopentényle, n-hexényle, isohéxényle, propargyle, n-butynyle, isobutynyle, n-pentynyle, isopentynyle, n-hexynyle, isohexynyle, chlorométhyle, bromométhyle, chloréthyle, brométhyle, chloropropyle, bromopropyle, cyanométhyle, cyanéthyle, cyanopropyle, hydroxyméthyle, hydroxyéthyle, hydroxypropyle, méthoxyméthyle, méthoxyéthyle, méthoxypropyle, éthoxyméthyle, éthoxyéthyle, éthoxypropyle, méthoxycarbonylméthyle, méthoxycarbonyléthyle, méthoxycarbonylpropyle, éthoxycarbonylméthyle, éthoxycarbonyléthyle, éthoxycarbonylpropyle, propoxycarbonylméthyle, propoxycarbonyléthyle, propoxycarbonylpropyle ; en outre, un groupe cyclopropyle, cyclopropylméthyle, cyclopropyléthyle, cyclopropylpropyle, cyclopentyle, cyclopentylméthyle, cyclopentyléthyle, cyclopentylpropyle, cyclohexyle, cyclohexylméthyle, cyclohexyléthyle ou cyclohexylpropyle portant chacun le cas échéant, dans la partie cycloalkyle, un à quatre substituants, identiques ou différents, fluor, chlore, brome, méthyle, éthyle, n-propyle, isopropyle, chlorométhyle, dichlorométhyle ou trifluorométhyle ; en outre, un groupe phénylalkyle, phénylalcényle, phénylalcynyle, phényle ou naphtyle portant chacun le cas échéant dans la partie aryle un à trois substituants identiques ou différents et ayant chacun le cas échéant jusqu'à 6 atomes de carbone dans la partie alkyle ou alcényle ou alcynyle linéaire ou ramifiée, en considérant dans chaque cas comme substituants de la partie aryle : le fluor, le chlore, le brome, un radical hydroxy, cyano, nitro, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, trifluorométhyle, trifluorométhoxy, trifluorométhylthio, méthylcarbonyle, éthylcarbonyle, méthoxycarbonyle, éthoxycarbonyle, aminocarbonyle, N-méthylaminocarbonyle, N,N-diméthylaminocarbonyle, N-éthylaminocarbonyle, N,N-diéthylaminocarbonyle, N-formylamino, N-acétylamino, N-méthyl-N-formylamino, N-méthyl-N-acétylamino, N-éthyl-N-formylamino, N-éthyl-N-acétyl-amino, méthoximinométhyle, méthoximinoéthyle, éthoximinométhyle, éthoximinoéthyle ou un groupe phényle portant le cas échéant un à trois substituants, identiques ou différents, fluoro, chloro, bromo, méthyle et/ou éthyle,
R est de l'hydrogène, un groupe méthyle, éthyle, n-propyle, isopropyle, n-butyle, isobutyle, sec.-butyle, tertiobutyle, n-pentyle, isopentyle, n-hexyle, isohexyle, hydroxyméthyle, hydroxyéthyle, hydropropyle, chlorométhyle, bromométhyle, chloréthyle, brométhyle, chloropropyle, bromopropyle, cyanométhyle, cyanéthyle, cyanopropyle, hydroxyméthyle, hydroxyéthyle, hydroxypropyle, méthoxyméthyle, méthoxyéthyle, méthoxypropyle, éthoxyméthyle, éthoxyéthyle, éthoxypropyle, méthoxycarbonylméthyle, méthoxycarbonyléthyle, méthoxycarbonylpropyle, éthoxycarbonylméthyle, éthoxycarbonyléthyle, éthoxycarbonylpropyle, propoxycarbonylméthyle, propoxycarbonyléthyle, propoxycarbonylpropyle, diméthylaminométhyle, diéthylaminométhyle, dipropylaminométhyle, diméthylaminoéthyle, diéthylaminoéthyle, dipropylaminoéthyle, diméthylaminopropyle, diéthylaminopropyle ou dipropylaminopropyle, ou bien
R¹ et R forment, conjointement avec l'atome d'azote auquel ils sont liés, un hétérocycle de formule 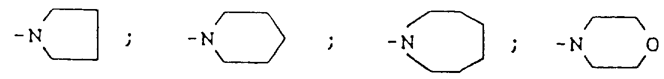 ou 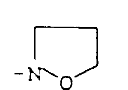 portant le cas échéant un, deux ou trois substituants méthyle et/ou éthyle, méthoxycarbonyle et/ou éthoxycarbonyle et
R³, R⁴, R⁵ et R⁶ représentent chacun, indépendamment les uns des autres, de l'hydrogène, du fluor, du chlore, du brome, un radical cyano, nitro, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, trifluorométhyle, trifluorométhoxy, ou trifluorométhylthio.

4. Procédé de production de S,S-dioxydes de benzothiophène-2-carboxamides de formule 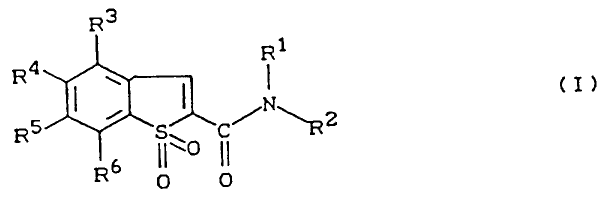 dans laquelle
R¹, R, R³, R⁴, R⁵ et R⁶ ont les définitions indiquées dans la revendication 1,
caractérisé en ce qu'on fait réagir des benzothiophène-2-carboxamides de formule 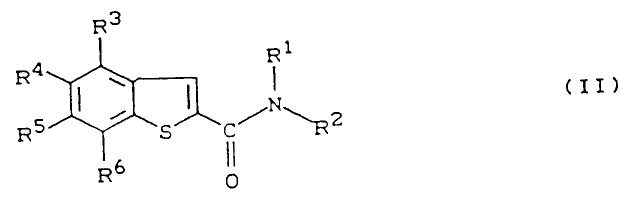 dans laquelle
R¹, R, R³, R⁴, R⁵ et R⁶ ont la définition indiquée ci-dessus,
avec un agent oxydant, le cas échéant en présence d'un diluant et en la présence éventuelle d'une substance auxiliaire de réaction.

5. Compositions pesticides, caractérisées par une teneur en au moins un S,S-dioxyde de benzothiophène-2-carboxamide de formule (I) suivant la revendication 1.

6. Utilisation de S,S-dioxydes de benzothiophène-2-carboxamides de formule (I) suivant la revendication 1 pour combattre des parasites dans le domaine de la protection des plantes et pour la protection des matériaux.

7. Procédé pour combattre des parasites dans le domaine de la protection des plantes et dans la protection des matériaux, caractérisé en ce qu'on applique des S,S-dioxydes de benzothiophène-2-carboxamides de formule (I) suivant la revendication 1 sur les parasites et/ou sur leur milieu.

8. Procédé de préparation de compositions pesticides, caractérisé en ce qu'on mélange des S,S-dioxydes de benzothiophène-2-carboxamides de formule (I) suivant la revendication 1 avec des diluants et/ou des agents tensioactifs.
